(19) Europäisches Patentamt
European Patent Office
Office européen des brevets

(11) Publication number: **0 159 784**
**B1**

(12) **EUROPEAN PATENT SPECIFICATION**

(45) Date of publication of patent specification: **07.06.89**

(21) Application number: **85301533.7**

(22) Date of filing: **06.03.85**

(51) Int. Cl.$^4$: **C 07 C 59/72,** C 07 C 43/21, C 07 D 307/92, C 07 D 317/72, A 61 K 31/557

(54) **Carbacyclin analogues.**

(30) Priority: **08.03.84 US 587336**
**08.03.84 US 587337**
**11.01.85 US 690803**
**11.01.85 US 690804**

(43) Date of publication of application:
**30.10.85 Bulletin 85/44**

(45) Publication of the grant of the patent:
**07.06.89 Bulletin 89/23**

(84) Designated Contracting States:
**BE CH DE FR GB IT LI NL SE**

(56) References cited:
**EP-A-0 069 692**
**EP-A-0 087 237**
**DE-A-2 834 372**
**GB-A-2 121 802**
**US-A-3 980 694**
**US-A-4 125 544**
**US-A-4 259 240**
**US-A-4 306 075**

**The file contains technical information submitted after the application was filed and not included in this specification**

(73) Proprietor: **THE UPJOHN COMPANY**
**301 Henrietta Street**
**Kalamazoo, Michigan 49001 (US)**

(72) Inventor: **Aristoff, Paul Adrian**
**c/o The Upjohn Company 301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

(56) References cited:
**Advances in Prostaglandin Thrombexane and Leukotriene Research, Vol.11, (1983), pages 267-274**

**CHEMICAL ABSTRACTS, vol. 95, no.3,10 July 20 1981Columbus Ohio, USA KENDE, ANDREW S.; BOETTGER, SUSAN D. "Regionspecific total synthesis of (=)-11-deoxycarminomycinone"page 654, column 2, abstract no. 24623t**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 159 784 B1**

(56) References cited:
CHEMICAL ABSTRACTS,vol. 97, no. 23, December 6, 1982, Columbus, Ohio, USA JEFFS, PETER W.; MOLINA, GERALDO; CASS, MALCOM W.,CORTESE, NICHOLAS A. "Sceletium alkaloids. part. 10. Synthesis of 1-substituted cisbyciclo (4.2.0)octanones through (2+2) cycloadditions of dichloroketene to alkenes. Structural characterization of cycloadducts by oxa-ring expansion", page 599, col. 2, abstract 198420f.

CHEMICAL ABSTRACT, vol, 95, no. 11, Sept.14, 1981,Columbus, Ohio, USA KOCOVSKY, PAVEL; CERNY, VACLAV "Steroids. CCXLIV. A modified route to intermediates in partial syntheses of digitoxigenin and xysmalogenin"; page 693, col. 1, abstract 98139m.

**Description**

Field of the Invention

The present invention relates to novel, pharmaceutically-useful compounds which are carbacyclin analogues having a tricyclic nucleus.

Prior Art

Background art on carbacyclin and related compounds is given in EP—A—0087237. Interphenylene carbacyclins are described in US—A—4306075, US—A—4306076 and EP—A—0087237. Compounds having a 5-membered oxa ring are described in EP—A—0024943. The utility and synthesis of compounds closely related to those of the present invention are described by Aristoff and Harrison in Tet. Lett. *23* :1982) 2067—2070, and in Advances in Prosglandin, Thromboxane and Leukotriene Research *11*(1983) 267. This last reference discloses the compound of formula I (see below) in which $R_1$ is H, $L_{20}$ is α—OH:β—H, $M_1$ is α—OH:β—H and R is cyclopentyl (as Compound No. 28).

Summary of the Invention

Novel compounds are of formula I,

wherein $R_1$ is H, a pharmacologically-acceptable cation, $C_{1-12}$ alkyl, $C_{3-10}$ cycloalkyl, $C_{7-12}$ aralkyl, phenyl optionally substituted by one, 2 or 3 Cl atoms or $C_{1-3}$ alkyl groups, or phenyl para-substituted by a substituent selected from —NHCOR$_{25}$, —COR$_{26}$, —OOC—R$_{54}$ and —CH=N—NH—CONH$_2$ wherein $R_{25}$ is methyl, phenyl, acetamidophenyl, benzamidophenyl or $NH_2$, $R_{26}$ is methyl, phenyl, methoxy or $NH_2$, and $R_{54}$ is phenyl or acetamidophenyl;

$L_{20}$ is α—OH:β—H, α—H:β—OH, H:H, α—$CH_3$:β—H, α—$CH_2OH$:β—H, O or $CH_2$;

$M_1$ is α—H:β—H, O, α—OH:β—$R_5$ or α—$R_5$:β—OH, $R_5$ being H or $CH_3$; and

R is $C_{4-7}$ cycloalkyl, 4-oxocyclohexyl, 4-tetrahydropyranyl or 2-tetrahydropyranyl;

provided always that $M_1$ is H:H or O and/or $L_{20}$ is α—$CH_3$:β—H, O or $CH_2$.

The compounds of formula I have useful pharmacological properties.

Other compounds of the invention, useful as intermediates, are of the formulae Id and Ie,

wherein $L_{22}$ is α—$OR_x$:β—H, α—H:β—$ORT_x$, H:H, α—$CH_3$:β—H, α—$CH_3$—β—H, O or $CH_2$; $R_a$ is $C_{4-7}$ cycloalkyl, 4-(ethylenedioxy)cyclohexyl, 4-oxocyclohexyl, 4-tetrahydropyranyl or 2-tetrahydropyranyl; $R_b$ is $C_{4-7}$ cyclalkyl, 4-(ethylenedioxy)cyclohexyl, 4-tetrahydropyranyl or 2-tetrahydropyranyl; $X_2$ is H or $CH_3$; and $M_2$ is H:H, ethylenedioxy, α—H:β—$OR_x$ or α—$OR_x$—β—H, $R_x$ being a protecting group;

provided always that (in formula Ie) $M_2$ is H:H and/or $L_{20}$ is α—$CH_3$:β—H, O or $CH_2$.

Description of the invention

In the compounds of the present invention, and as used herein, (''') denotes the α- configuration, (—) denotes the β- configuration, (∾) denotes α- and/or β- configuration or the E and/or Z isomer.

With regard to the divalent groups described above, i.e. $L_{20}$, $L_{22}$, $M_1$ and $M_2$, they are defined in terms of an α-substituent and a β-substituent which means that the α-substituent of the divalent group is in the alpha configuration with respect to the plane of the C—8 to C—12 cyclopentane ring and the β-substituent is in the beta configuration with respect to the cyclopentane ring.

In Formula I when the hydrogen at position 9 is beta the compounds are named as 9-deoxy-13,14-dihdro-2′, 9α-methano-3-oxa-4,5,6-trinor-3,7-(1′,3′-interphenylene)-PGF$_1$ compounds, and when it is alpha the compounds are named as 9-deoxy-13,14-dihydro-2′,9β-methano-3-oxa-4,5,6-trinor-3,7-(1′,3′-interphenylene)PGF$_1$ compounds.

When $R_5$ is methyl, the carbacyclin analogs are all named as "15-methyl-" compounds. Further, compounds wherein the $M_1$ moiety contains an hydroxyl in the beta configuration are additionally named as "15-epi-" compounds.

Compounds wherein $M_1$ is H,H are named as "15-deoxy" compounds. Compounds wherein $M_1$ is =O are named as "15-oxo" compounds.

Wherein $R_1$ is other than hydrogen the novel compounds herein are named as esters and salts.

Examples of phenyl esters substituted in the para position (i.e. $R_1$ is p-substituted phenyl) include p-acetamidophenyl ester, p-benzamidophenyl ester, p-(p-acetamidobenzamido)phenyl ester, p-(p-benzamidobenzamido)phenyl ester, p-amidocarbonylaminophenyl ester, p-acetylphenyl ester, p-benzoylphenyl ester, p-aminocarbonylphenyl ester, p-methoxycarbonylphenyl ester, p-benzoyloxyphenyl ester, p-(p-acetamidobenzoyloxy)phenyl ester, and p-hydroxybenzaldehyde semicarbazone ester.

Examples of $C_{1-12}$ alkyl are methyl, ethyl, propyl, isopropyl, isobutyl, tert-butyl, isopentyl, neopentyl, butyl, pentyl, nexyl, heptyl, octyl, nonyl, decyl, undecyl, dodecyl isomeric forms thereof.

Examples of $C_{3-10}$ cycloalkyl, which includes alkyl-substituted cycloalkyl, are cyclopropyl, 2-mehylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 2-pentylcyclopentyl, 3-tert-butylcyclopentyl, cyclohexyl, 4-tertbutylcyclohexyl, 3-isopropylcyclohexyl, 2,2-dimethylcyclohexyl, cycloheptyl, cyclooctyl, cyclononyl, and cyclodecyl.

Examples of $C_{7-12}$ aralkyl are benzyl, 2-phenylethyl, 1-phenylethyl, 2-phenylpropyl, 4-phenylbutyl, 3-phenylbutyl, 2-(1-naphthylethyl), and 1-(2-naphthylmethyl).

Examples of phenyl substituted by one to 3 Cl atoms or $C_{1-3}$ alkyl groups are p-chlorophenyl, m-chlorophenyl, 2,4-dichlorophenyl, 2,4,6-trichlorophenyl, p-tolyl, m-tolyl, p-tolyl, p-ethylphenyl, 2,5-dimethylphenyl, 4-chloro-2-methylphenyl, and 2,4-dichloro-3-methylphenyl.

The compounds of Formula I produce certain prostacyclin-like pharmacological responses. Accordingly, the Formula I compounds are useful as agents in the study, prevention, control, and treatment of diseases, and other undesirable physiological conditions, in mammals, particularly humans, valuable domestic animals, pets, zoological specimens, and laboratory animals (e.g., mice, rats, rabbits and monkeys). In particular, these compounds are useful as anti-ulcer agents, and anti-asthma agents, and as antithrombotic agents as indicated below.

(a) Platelet Aggregation Inhibition

The compounds of Formulas I are useful whenever it is desired to inhibit platelet aggregation, to reduce the adhesive character of platelets, or to remove or prevent the formation of thrombi in mammals, including man. For example, these compounds are useful in the treatment and prevention of myocardial infarcts, to treat and prevent post-operative thrombosis, to promote patency of vascular grafts following surgery, to treat peripheral vascular diseases, and to treat conditions such as atherosclerosis, arteriosclerosis, blood clotting defects due to lipemia, and other clinical conditions in which the underlying etiology is associated with lipid imbalance or hyperlipidemia. Other in vivo applications include geriatric patients to prevent cerebral ischemic attacks and long term prophylaxis following myocardial infarcts and strokes. For these purposes, these compounds are administered systemically, e.g., intravenously, subcutaneously, intramuscularly, and in the form of sterile implants for prolonged action. For rapid response, especially in emergency situations, the intravenous route of administration is preferred.

The preferred dosage route for these compounds is oral, although other non-parenteral routes (e.g. buccal, rectal, sublingual) are likewise employed in preference to parenteral routes. Oral dosage forms are conventionally formulated as, e.g. tablets or capsules and administered 2-4 times daily. Doses in the range of 0.05 to 100 mg per kg of body weight per day are effective in treating the aforedescribed conditions associated with the inhibition of platelet aggregation. Doses in the range 0.01 to 10 mg per kg of body weight per day are preferred, the exact dose depending on the age, weight, and condition of the patient or animal, and on the frequency and route of administration.

The addition of these compounds to whole blood provides in vitro applications such as storage of whole blood to be used in heart-lung machines. Additionally whole blood containing these compounds can be circulated through organs, e.g., heart and kidneys, which have been removed from a donor prior to transplant. They are also useful in preparing platelet rich concentrates for use in treating thrombocytopenia, chemotherapy, and radiation therapy. In vitro applications utilize a dose of 0.001-1.0 μg per ml of whole blood. The compounds of the present invention are useful in the treatment of peripheral vascular diseases, in the same manner as described in US—A—4,103,026.

(b) Gastric Secretion Reduction

Compounds of Formulas I are useful in mammals, including man and certain useful animals, e.g., dogs and pigs, to reduce and control gastric secretion, thereby to reduce or avoid gastrointestinal ulcer formation, and accelerate the healing of such ulcers already present in the gastrointestinal tract. For this purpose, these compounds are injected or infused intravenously, subcutaneously, or intramuscularly in an infusion dose range of 0.1 to 20 μg per kg of body weight per minute, or in a total daily dose by injection or infusion in the range 0.01 to 10 mg per kg of body weight per day, the exact dose depending on the age, weight, and condition of the patient or animal, and on the frequency and route of administration.

Preferably, however, these novel compounds are administered orally or by other non-parenteral routes. As employed orally, one to 6 administrations daily in a dosage range of 0.001 to 100 mg per kg of body weight per day is employed. Once healing of the ulcers has been accomplished the maintenance dosage required to prevent recurrence is adjusted downward so long as the patient or animals remains asymptomatic. The compound of specific Example 2 below demonstrates good cytoprotective properties with very low blood pressure effects in the rat and thus represents a preferred compound of the present invention.

The final products of specific Examples 4 and 5 below demonstrate good cytoprotective properties with relatively low blood pressure effects in rats rendering said compounds preferred embodiments of the present invention.

(c) NOSAC-Induced Lesion Inhibition

Compounds of Formulas I are also useful in reducing the undesirable gastrointestinal effects resulting from systemic administration of anti-inflammatory prostaglandin synthetase inhibitors, and are useful for that purpose by concomitant administration of said compounds of Formulas I and the anti-inflammatory prostaglandin synthetase inhibitor. See US—A—3,781,429 for a disclosure that the ulcerogenic effect induced by certain non-steroidal anti-inflamatory agents in rats is inhibited by concomitant oral administration of certain prostaglandins of the E series. Accordingly these novel Formula I compounds are useful, for example, in reducing the undesirable gastrointestinal effects resulting from systemic administration of known prostaglandin synthetase inhibitors, e.g., indomethacin, phenylbutazone, and

aspirin, in the same manner as described for the PGE compounds in US—A—3,781,429.

The anti-inflammatory synthetase inhibitor, for example, indomethacin, aspirin, or phenylbutazone is administered in any of the ways known in the art to alleviate an inflammatory conditions, for example, in any dosage regimen and by any of the known routes of systemic administration.

(d) Bronchodilation (Anti-asthma)

The compounds of Formulas II are also useful in the treatment of asthma. For example, these compounds are useful as bronchodilators or as inhibitors of mediator-induced bronchoconstriction, such as SRS—A, and histamine which are released from cells activated by an antigen-antibody complex. Thus, these compounds control spasm and facilitate breathing in conditions such as bronchial bronchitis, bronchiectasis, pneumonia and emphysema. For these purposes, these compounds are administered in a variety of dosage forms, e.g., orally in the form of tablets, capsules, or liquids; rectally in the form of suppositories, parenterally, subcutaneously, or intramuscularly, with intravenous administration being preferred in emergency situations; by inhalation in the form of aerosols or solutions for nebulizers; or by insufflation in the form of powder. Doses in the range of 0.01 to 5 mg per kg of body weight are used 1 to 4 times a day, the exact dose depending on the age, weight, and condition of the patient and on the frequency and route of administration. For the above use Formula I compounds can be combined advantageously with other anti-asthmatic agents, such as sympathomimetics (isoproterenol, phenylephrine, ephedrine, etc.); xanthine derivatives (theophylline and aminophylline); and corticosteroids (ACTH and prednisolone).

The pharmacologically useful Formula I compounds are effectively administered to human asthma patients by oral inhalation or by aerosol inhalation. For administration by the oral inhalation route with conventional nebulizers or by oxygen aerosolization it is convenient to provide the instant active ingredient in dilute solution, preferably at concentrations of about one part of medicament to from 100 to 200 parts by weight of total solution. Entirely conventional additives may be employed to stabilize these solutions or to provide isotonic media, for example, sodium chloride, sodium citrate, citric acid, sodium bisulfite, and the like can be employed. For administration as a self-propelled dosage unit for administering the active ingredient in aerosol form suitable for inhalation therapy the composition can comprise the active ingredient suspended in an inert propellant (such as a mixture of dichlorodifluoromethane and dichlorotetrafluoroethane) together with a col-solvent, such as ethanol, flavoring materials and stabilizers. Suitable means to employ the aerosol inhalation therapy technique are described fully in US—A—3,868,691, for example.

The novel Formula I compounds so described are used for the purposes described above in the free acid form, in ester form, or in pharmacologically acceptable salt form. When the ester form is used, the ester is any of those within the above definition of $R_1$. However, it is preferred that the ester be alkyl of one to 12 carbon atoms. Of the alkyl esters, methyl and ethyl are especially preferred for optimum absorption of the compound by the body or experimental animal system; and straight-chain octyl, nonyl, decyl, undecyl, and dodecyl are especially preferred for prolonged activity.

Pharmacologically acceptable salts of the novel compounds of Formula I for the purposes described above are those with pharmacologically acceptable metal cations, ammonia, amine cations, or quaternary ammonium cations. Illustrative pharmacoloigcal acceptable cations which $R_1$ may represent are the following.

Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amine cations are those derived from primary, secondary, and tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, adamantylamine, and the like aliphatic, cycloaliphatic, araliphatic amines containing up to and including about 18 carbon atoms, as well as heterocyclic amines, e.g., piperidine, morpholine, pyrrolidine, piperazine, and lower-alkyl derivatives thereto, e.g., 1-methylpiperidine, 4-ethylmorpholine, 1-isopropylpyrrolidine, 2-methylpyrrolidine, 1,4-dimethylpiperazine, 2-methylpiperidine, and the like as well as amines containing water-solubilizing or hydrophilic groups, e.g., mono-, di-, and triethanolamine, ethyldiethanolamine, N-butylethanolamine, 2-amino-1-butanol, 2-amino-2-ethyl-1,3-propanediol, 2-amino-2-methyl-1-propanol, tris(hydroxymethyl) aminomethane, N-phenylethanolamine, N-(p-tert-amylphenyl)-diethanolamine, galactamine, N-methylglycamine, N-methylglucosamine, ephedrine, phenylephrine, epinephrine, procaine, and the like. Further useful amine salts of the basic amino acid salts, e.g., lysine and arginine.

Examples of suitable pharmacologically acceptable quaternary ammonium cations are tetramethylammonium, tetraethylammonium, benzyltrimethylammonium, phenyltriethylammonium, and the like.

To obtain the optimum combination of biological response specificity, potency, and duration of activity, certain compounds within the scope of this invention are preferred. Preferred compounds of the present invention are Formula I compounds wherein $R_1$ is hydrogen, methyl, ethyl or a pharmacologically acceptable cation such as sodium.

In describing the preparation of the compounds of the present invention, reference is made to Chart A to Chart H. In the Charts, the various substituent groups have the following meanings: R, $R_a$, $R_b$, $L_{20}$, $L_{22}$, $M_1$ and $M_2$ are as defined above; alkyl is $C_{1-4}$ hydrocarbyl, straight or branched, e.g. methyl or ethyl; $L_{21}$ is α-$OR_x$:β-H, α-H:β-$OR_x$, H:H, α-$CH_3$:β-H, α-$CH_2OR_x$:β-H or $CH_2$; and $L_{22}$ is $L_{21}$ or O. $W_2$ is as defined in Chart B. Ph is phenyl.

For the purposes of protecting the various hydroxyl groups at positions 11, 15 and 16, many suitable protecting groups $R_x$ are known; for example, they and the manner of introducing and removing them are described in US—A—4401824, particularly column 11, line 21 to column 13, line 15. Although any of these protecting groups may be employed, those preferred are tetrahydropyranyl, tetrahydrofuranyl, tert-butyldimethyl- silyl and tert-butyldiphenylsilyl. It may be useful, of course, to use protecting groups which may be hydrolysed selectively.

The compounds of the present invention are prepared by various means utilising 2,3,3a,4-tetrahydro-5-methoxy-2-oxo-naphtho[2,3-b]furan (depicted as Formula II in Charts C and D). Although, in describing the preparation of the compounds of Formula I only one optical enantiomer may be depicted, the processes are applicable to both the D and L optical isomers or mixtures thereof unless, of course, a particular step is stereoselective.

The compounds of Formula I are prepared as depicted in Chart A. The enol-lactone (II) is alkylated with two equivalents of a phosphonate anion (III) followed by one equivalent of acetic acid after which the reaction is warmed to effect the intramolecular Wadsworth-Emmons reaction, this procedure being an improved modification of the procedure of C. A. Henrick *et al*, J. Am. Chem. Soc. *90*, 5926 (1968). The resulting enone (IV) is reduced to the ketone (V) by procedures known in the art. For example, the enone is hydrogenated over palladium catalyst in ethanol at 3 atmospheres pressure and this may be followed by oxidation if necessary using, for example, Jones reagent. Equilibration to the thermodynamically favored ketone is achieved typically under basic conditions using, for example, potassium hydroxide in ethanol by procedures known in the art.

To prepare intermediates (VI) wherein $L_{21}$ is α-H,β-OH or α-OH,β-H, the ketone (V) is reduced by procedures known in the art, for example using sodium borohydride. Conversion of the ketone (V) to the intermediate (VI) where $L_{21}$ is methylene, i.e. $=CH_2$, typically is achieved via a Wittig-type procedure, for example, using methylenetriphenylphosphorane by generally known procedures. Alternatively, the methylene group can be prepared by treatment of ketone (V) with the anion of methylphenyl-N-methylsulfoxime in tetrahydrofuran followed in a subsequent step by sulfoxime elimination with aluminium amalgam (see Aristoff, P. A. and Harrison, A. W., Tetrahedron Letters 23l, 2067—2070 (1982)). The methylene intermediate can be used to prepare compounds IX as depicted in Chart A or can be reduced to the corresponding compound wherein $L_{21}$ is α-$CH_3$,β-H, for example, via hydrogenation over palladium catalyst by procedures known in the art. The methylene intermediate can also be used to prepare the corresponding compound wherein $L_{21}$ is α—$CH_2$,β-H by hydroboration using, for example, 9-borobicyclononane (9-BBN) followed by work-up with basic hydrogen peroxide. The intermediates of formula (VI) wherein $L_{21}$ is H, H are prepared by treating the ketone (V) with a hydrazine derivative, such as tosylhydrazine, followed by a Shapiro reaction on the resulting tosylhydrazone (see R. H. Shapiro, Chapter 3 in Organic Reactions, Volume 23, pp. 405—507); the 10,11-didehydro intermediate thus obtained in hydrogenated, e.g. using palladium over charcoal.

Following the various conversions at the 11-position any ketal of $R_b$ can be hydrolyzed, typically with aqueous acid which will also remove any hydroxyl protecting groups in $M_2$ or hydrolyze the $M_2$ ketal. The $M_2$ hydroxyl groups can be reprotected at this point if necessary.

The compounds of (V) and (VI) are converted to the phenols (VII) by, for example, treatment with lithium diphenylphosphide in tetrahydrofuran as generally described by R. E. Ireland and D. M. Walba, Tetrahedron Letters, 1071 (1976). Other methods for aryl methyl ether cleavages are known and may be employed, e.g. see M. V. Bhatt and S. U. Kulharni, Synthesis 249 (1983). The phenols are converted to acids (VIII: $R_1$=H) using, for example, potassium carbonate and chloromethyl nitrile, followed by hydrolysis. The phenols are converted to alkyl esters (VIII) by treatment with one equivalent of base, e.g. sodium hydride, and an appropriate halo alkanoate, e.g. a $C_{1-4}$ alkyl bromoacetate. The esters are hydrolysed to the corresponding carboxylic acids by procedures known in the art, for example, by using aqueous potassium hydroxide in methanol. The carboxylic acids are converted to the final products (IX) wherein $R_1$ is H upon hydrolysis of any protecting groups at positions 11, 15 or 16 and the ketal protecting the C-15 as carbonyl. The carboxylic acid derivative also can be converted to the various esters defined by Formula I.

Compounds of Formula I may also be prepared using the aldehyde depicted in Chart B as Formula XI. By the procedures generally described in Chart U of US—A4306075, the Formula XI aldehyde is reacted with an alkyl phosphonate of Formula X under the conditions of a Wittig reaction to give a ketone of Formula XII. The ketone is reduced by hydride reduction to the trans-vinyl α- or β-alcohol, i.e. compounds of formula XIII wherein $M_3$ is α-OH,β-H or α-H,β-OH. The trans-vinyl alcohol of XIII is hydrogenated to give a compound of Formula XIV. When $R_5$ in the $M_1$ substituent of Formula I is methyl the appropriate starting material is obtained by oxidising the alcohol of Formula XIV to the corresponding ketone by procedures known in the art, and then treating the resulting ketone with methyl lithium or a methyl Grignard by well known procedures. The compounds of Formulas XIII and XIV wherein $M_3$ is α-H,β-OH or α-OH,β-H can be treated with a leaving group, e.g. converting the $M_3$=OH group to OTs followed by a displacement reaction,

e.g. with lithium aluminium hydride, to give the corresponding compounds wherein $M_3$ is H,H.

The compounds of Formula XIV are converted to final products of Formula I or by the same procedures set forth in Chart A for converting compounds VI and V to compounds IX. Prior to making these conversions any hydroxyl groups at positions 11, 15, 16 or in the R group can be protected as ORx as described hereinabove.

The compounds of Formula XI are prepared as set forth in Chart C and Chart D. In Chart C the 2,3,3A,4-tetrahydro-5-methoxy-2-oxo-naphtho[2,3-b]furan (Formula II) is treated with the anion of trimethylphosphonoacetate followed by cyclization as generally described in connection with the reaction of compounds of Formulas II and III in Chart A. Alternatively the lactone (II) is treated at low temperature with the anion of methyl acetate (or ethyl acetate to give the ethyl ester analog) followed by warming to effect the cyclization. Compounds XX are reduced to the ketone XXI by means known in the art, e.g., by hydrogenation using palladium catalyst. The ketone XXI is reduced to the C-11 alcohol by, e.g., treatment with sodium borohydride after which the carboxy ester is reduced to the hydroxymethyl compound of formula XXII using, e.g., excess diisobutylaluminum hydride. The compound of formula XXII is converted to the aldehyde of formula XI by procedures known in the art, e.g., by protection of the C-13 alcohol with an ORx group followed by oxidation of the C-11 alcohol to the ketone, e.g., with Collins reagent, followed by conversion of the C-11 ketone to any of the $L_{22}$ groups as previously described, hydrolysis of the C-13 protecting group and oxidation to the aldehyde.

In Chart D the lactone II is alkylated with the anion of dimethylphosphonate in a manner similar to that described for the reaction of compounds II and III in Chart A. The enone of Formula XXIII is reduced, e.g., by hydrogenation at room temperature over palladium catalyst by procedures known in the art to give the ketone of Formula XXIV the ketone enolate of which is alkylated using benzylchloromethyl ether by procedures well known in the art to give compounds of Formula XXV wherein ph is phenyl. The ketones of Formula XXV are converted to the various C-11 analogs of Formula XXVI by the same general procedures described for the conversion of compounds V to compounds VI in Chart A. Any hydroxyl group present at the C-11 substituent is protected appropriately as described hereinbefore prior to proceeding to compounds of Formula XXVII. Cleavage of the benzyl ethers of Formula XXVI by hydrogenation, procedures known in the art, gives the 12-hydroxymethyl compounds of Formula XXVII which are oxidized to the aldehydes of Formula XI using Collins reagent by known procedures.

The compounds of Formula IV in Chart A can also be prepared as depicted in Chart F. The 2,2-ethylenedioxy-5-methoxynaphthalen-3-ylacetic acid (Formula XXXVI) is reacted with two equivalents of a phosphonate of Formula III as generally described in connection with the preparation of compounds of Formula IV in Chart A to give the compounds of Formula XXXVII. The Formula XXXVII compound is deketalized by means known in the art, e.g., by treatment with aqueous acid followed by reprotection of any hydroxyl groups in $M_2$ chain as generally described herein. The ketone of Formula XXXVIII is then treated with base, e.g., sodium hydride in glyme to give the enone of Formula IV.

Compounds of Formula I can also be prepared as depicted in Chart E. Compounds XXVIII are obtained as depicted in Chart D (see compounds XXV and XXVI) and are converted to the phenols of Formula XXIV by cleavage of the methyl ether using lithium diphenylphosphide in tetrahydrofuran as generally described hereinabove in connection with the preparation of compounds VII in Chart A. The phenols of Formula XXIX are converted to the compounds of XXX by the general procedures described in connection with the compounds of Formula VII to compounds of Formula IX in Chart A. The compounds of Formula XXX are converted to the aldehydes of Formula XXXIV by the general procedures described in connection with the preparation of compounds XI from compounds XXVI in Chart D, and the aldehydes of Formula XXXIV in turn are converted to the compounds of Formula XXXV by the general procedures described in Chart B for preparing compounds XIX.

Compounds of Formula I also can be prepared as depicted in Chart E beginning with compounds of Formula XXXI which are obtained as described in Chart C (see Formulas XXI and XXII). Cleavage of the methyl ether of XXXI is accomplished using lithium diphenylphosphide in tetrahydrofuran as described hereinbefore followed by acid esterification, e.g., with diazomethane, and the resulting phenols (XXXII) are converted to the compounds of Formula XXXIII by the general procedures described in connection with the conversion of compounds VII to compounds IX in Chart A. The compounds of XXXIII are then converted to the corresponding aldehydes of XXXIV as generally described in Chart C (i.e., XXII to XI).

The phosphonates of Formulas III (Chart A) and X (Chart B) are known in the art or are prepared by procedures known in the art (see, for example, US—A—4,029,681 and US—A—4,401,824). Additionally in Charts G to I methods for obtaining appropriate phosphonates are described.

Chart G depicts the preparation of the appropriate phosphonate useful in obtaining compounds of Formula I wherein $M_1$ is H, H and R is cyclohexyl.

In Chart G, cyclohexanone (*1*) is reacted with 3-carboxypropyltriphenylphosphorane under conditions of a Wittig reaction by known procedures. The resulting acid (*2*) is reduced to the alcohol (*3*) by known procedures, e.g., using lithium aluminum hydride. The alcohol is converted to the corresponding halide using, e.g., triphenylphosphine and carbon tetrachloride to give compound *4a* or by tosylation followed by tosylate displacement with sodium iodide to give compound *4b* by known procedures. The halide is treated with trimethylphosphite under conditions of an Arbuzoz reaction by known procedures to give the phosphonate *5* the use of which in preparing compounds of Formula I is further depicted in Chart G. The

anion of compound *5* is formed, e.g., by treatment with butyl lithium, and the anion is reacted with compound II (Chart A) in the same manner as compounds III and II are reacted in Chart A. The resulting enone *6* can be reduced to the alcohol, compound *7*, by the procedures described in connection with converting compound of Formula IV to compounds of Formula VI wherein $L_{21}$ is hydroxyl in Chart A.

It is readily apparent that the various C-11 position conversions described in connection with compounds of Formula V to give compounds of Formula VI in Chart A may be performed in the same manner on the intervening C-11 ketone formed upon reduction of the enone. Compound *7* is then treated in a manner as described in connection with compounds of Formula VI (Chart A) to obtain compounds of Formula I. By substituting an appropriate cycloalkanone for cyclohexanone other compounds of Formula I are obtained.

In Chart H the aldehyde (*11*) is alkylated with vinyl Grignard or vinyl lithium to give the vinyl alcohol (*15*) by procedures known in the art. Kinetic resolution of the vinyl alcohol (*15*) to give compound (*16*) is accomplished by the method of Sharpless (Martin, V. S., et al., J. Am . Chem. Soc. *103,* 6237 (1981). Alternatively, the aldehyde is alkylated with acetylene anion by known procedures to give the ethynyl alcohol (*12*) which is oxidized to the ketone (*13*) using, e.g., Jones reagent, by known procedures. The ketone (*13*) is reduced assymetrically using chiral reagents by known procedures. See, e.g., Midland, M. M., J. Org. Chem. *47,* 2815 (1982); J. Org. Chem. *46,* 3933 (1981), and J. Am. Chem. Soc. 102, 867 (1980); and also, Cohen, N., J. Org. Chem. *45,* 583 (1980) and Brinkmeyer, R. S., and Kapoor, V., J. Am. Chem. Soc. *99,* 8339 (1977). The ethynyl alcohol (*14*) is then partially reduced using, e.g., sodium bis(2-methoxyethoxy) aluminum hydride in toluene or hydrogenation over a Lindlar catalyst by known procedures. The vinyl alcohol (*16*) is then protected, e.g., as a tetrahydropyranyl group and either subjected to iodoboration by the general procedures of H. C. Brown, "Organic Synthesis via Boranes," John Wiley, N.Y., 1975, pp. 101—102, to give compound (*21*) or is subjected to hydroboration and oxidation using, e.g., 9-borabicyclononane followed by alkaline peroxide work-up by known procedures to give 3-$R_{71}$-3-ORx-propanol. The propanol is converted to compound *21* by direct replacement of the primary OH with iodide using iodine and a triaryl phosphine as generally described by B. R. Castro, "Organic Reactions," *29,* p. 1, ed., W. G. Dauben, John Wiley, N.Y., 1983. Alternatively the primary OH of the propanol is selectively activated, e.g., via tolsylation, followed by displacement of the tosylate with iodide in acetone and diisopropylamine to give compound (*21*). Compound (*21*) is treated with the anion of dialkyl methyl phosphonate to give compound (*22*).

The various aldehydes, compounds (*11*), utilized in Chart H are known in the art or are obtained by means known in the art. Illustratively, 4-tetrahydropyrancarboxaldehyde is obtained by ketone homologation of 4-tetrahydropyranone, e.g., using methoxymethyltriphenylphosphorane followed by aqueous acid enol ether hydroloysis by procedures known in the art. 2-Tetrahydropyrancarboxaldehyde is obtained by oxidizing 2-tetrahydropyranylmethanol using e.g., oxalyl chloride and dimethyl sulfoxide in methylene chloride by known procedures. In the case when R is 4-oxocyclohexyl, one of the carbonyl gorups of cyclohexane-1,4-dione is protected as a ketal following procedures known in the art and the remaining carbonyl is subjected to homologation as generally described hereinabove.

In Chart I the cyclohexylcarboxaldehyde (*1*) is alkylated with vinyl Grignard or vinyl lithium to give the vinyl alcohol (*5*) by procedures known in the art. Kinetic resolution of the vinyl alcohol (*5*) to give compound (*6*) is accomplished by the method of Sharpless (Martin, V. S., et al, J. Am. Chem. Soc. *103,* 6237 (1981). Alternatively, the cyclohexylcarboxaldehyde is alkylated with acetylene anion by known procedures to give the ethytnyl alcohol (*2*) which is oxidized to the ketone (*3*) using, e.g., Jones reagent, by known procedures. The ketone (*3*) is reduced asymmetrically using chiral reagents by known procedures. See, e.g., Midland, M. M., J. Org. Chem. *47,* 2815 (1982); J. Org. Chem. *46,* 3933 (1981), and J. Am. Chem. Soc., 102, 867 (1980); and also, Cohen, N., J. Org. Chem. *45,* 583 (1980) and Brinkmeyer, R. S., and Kapoor, V., J. Am. Chem. Soc. *99,* 8339 (1977). The ethynyl alcohol (*4*) is then partially reduced using, e.g., sodium bis(2-methoxyethoxy) aluminum hydride in toluene or hydrogenation over Lindlar catalyst by known procedures. The vinyl alcohol (*6*) is then protected, e.g., as a tetrahydropyranyl group, and either subjected to iodoboration by the general procedures of H. C. Brown, "Organic Synthesis via Boranes," John Wiley, N.Y., 1975, pp. 101—102, to give compound (*11*) or is subjected to hydroboration and oxidation using, e.g., 9-borabicyclononane followed by alkaline peroxide work-up by known procedures to give 4-cyclohexyl-4-ORx-propanol. The propanol is converted to compound *11* by direct replacement of the primary OH with iodide using iodine and a triaryl phosphine as generally described by B. R. Castro, "Organic Reactions," *29,* p. 1, ed., W. G. Dauben, John Wiley, N.Y., 1983. Alternatively the primary OH of the propanol is selectively activated, e.g., via tolsylation followed by displacement of the tosylate with iodide in acetone and diisopropylamine to give compound (*11*). Compound (*11*) is treated with the anion of dialkyl methyl phosphonate to give compound (*12*).

Although Chart I shows the preparation of cyclohexyl phosphonates, the same methods are applicable generally to phosphonates used herein.

Optically pure compounds of Formula I wherein $L_{20}$ is α-OH:β-H are obtained by attaching a chiral ester or chiral carbamate, e.g. (+)-α-methoxy-α-trifluoromethylphenyl acid chloride or (+)-α-methylbenzyl isocyanate, to a compound of Formula VI depicted in Chart A using procedures known in the art, giving a product which permits separation of the diastereomers. Following separation of the diastereomers, the compounds are subjected to ester hydrolysis by well known procedures, e.g. using potassium carbonate in

methanol or potassium hydroxide in water and methanol. The resolved diasteromers are then converted to the final products of the invention as depicted in Chart A proceeding from compounds of Formula VI to compounds of Formula IX. Alternatively, these optically pure compounds are obtained by reduction of the ketone depicted as Formula IV in Chart A to the corresponding alcohol i.e., 11-OH compound, by procedures well known in the art, e.g., using sodium borohydride in ethanol, followed by attachment of a chiral ester or a chiral carbamate as described above with subsequent separation of the diastereomers and ester hydrolysis. The resulting resolved diastereomers are then oxidized using e.g., manganese dioxide, or dimethylsulfoxide and dicyclohexylcarbodiimide to give a resolved ketone corresponding to Formula IV in Chart A which when treated as described in Chart A in going from Formula IV through Formula IX gives resolved compounds of the invention. Additionally, compounds of Formula I or I(a) wherein $L_{20}$ is OH, H can be resolved by e.g., treatment with (+)-α-methylbenzylamine by procedures known in the art to give a chiral amide followed by amide hydrolysis using, e.g., potassium hydroxide in water and methanol at reflux.

## Example 1

### 2,3,3A'-4-Tetrahydro-5-methoxy-2-oxo-naphtho[2,3-b]furan

(a) 3,4-Dihydro-2-hydroxy-5-methoxynaphthalenecarboxylic acid methyl ester

A solution of 5-methoxy-β-tetralone (20.6 g, 117 mmol) and 350 ml of dimethylcarbonate was cooled to 0 to 5°, then treated with 32 ml (140 mmol) of 25% sodium methoxide in oxygen-free methanol. The resulting dark brown solution was stirred for 30 minutes at 0°, then heated to 70°, stirred for 18 hours under a nitrogen atmosphere, then cooled to 0 to 5° and quenched with 200 ml of cold 1N degassed aqueous hydrochloric acid. The solution was extracted with ethyl acetate (2 × 150 ml). The combined organic layers were washed with brine (2 × 200 ml), dried over magnesium sulfate, filtered, and rotary evaporated at 50°. The resulting red-brown oil was crystallized from 80 ml of 1:1 ether/hexane in the freezer to give 14.43 g (53%) of yellow crystals, m.p. 56—58°. A second crop of yellow crystals (3.6 g, 14%) can be obtained from 20 ml 1:1 ether/hexane, m.p. 55—58°. The mother liquor (~12 g) was chromatographed on 100 g of silica gel 60 slurry packed in 300 ml of hexane. Eluting with 2% ethyl acetate in hexane gave 5.1 g (19%) of the title compound (a) in fractions 17—28, m.p. 53—58°. Total yield of compound (a) was 23.1 g (85%).

NMR ($CDCl_3$, TMS): δ 2.3—2.7 (m, 2H), 2.8—3.0 (m, 2H), 3.80 (s, 3H), 3.90 (s, 3H), 6.6—7.5 (m, 3H), 13.35 (s, 1H).

Infrared: $\nu_{max}$ (mull): 1640, 1598, 1587, 1566, 1422, 1378, 1311, 1277, 1220, 1207, 1086, 1052, 1030, 892, 787, 769, 721 $cm^{-1}$.

TLC (Silica Gel GF): Rf = 0.47 in 10% ethyl acetate in hexane.

(b) 3,4-Dihydro-2-hydroxy-3-(3-propene)-5-methoxynaphthalenecarboxylic acid methyl ester

A solution of 300 ml of tetrahydrofuran and 39 ml (282 mmol) of diisopropylamine under nitrogen was cooled to −50°C and treated with 170 ml (272 mmol) of 1.6M n-butyllithium in hexane dropwise maintaining the temperature at −50°C. The solution was stirred at −50° for 15 minutes, then at 0° for 15 minutes. A solution of 30.0 g (128.1 mmol) of 3,4-dihydro-2-hydroxy-5-methoxynaphthalenecarboxylic acid methyl ester in 70 ml of tetrahydrofuran was added dropwise to maintain the temperature at 0°. The resulting yellow suspension was treated with 13.5 ml (160 mmol) of allyl bromide in 50 ml of tetrahydrofuran dropwise maintaining the temperature at 0°. The cooling bath was removed and the orange solution was stirred at ambient temperature for 1 hour, then cooled to 10 to 15°C and 500 ml of 1N degassed aqueous hydrochloric acid was added dropwise maintaining the temperature below 15°. The layers were separated and the aqueous layer extracted with 400 ml of ethyl acetate. The organic layers were combined and washed with 500 ml of brine, dried over anhydrous magnesium sulfate, filtered and concentrated via rotary evaporation and then house vacuum to give 44.2 g of the title compound (b), m.p. 70—71°.

NMR ($CDCl_3$, TMS): δ 1.8—3.2 (m, 5H), (3H, singlets at 3.80 δ and 3.90 δ; 6H) 4.7—5.4 (m, 2H), 5.5—6.1 (m, 1H), 6.5—7.6 (m, 3H), 13.4 (s, 1H).

Infrared: $\nu_{max}$ 2925, 2956, 1237, 1598, 1440, 1270, 1257, 1051, 1002, 885, 790 772 $cm^{-1}$.

TLC (Silica Gel GF): Rf = 0.34 in 10% ethyl acetate in hexane.

(c) 1,2,3,4-Tetrahydro-5-methoxy-3-(3-propene)naphthalen-2-one

A mixture of 44.1 g of 3,4-dihydro-2-hydroxy-5-methoxy-3-(3-propene)naphthalenecarboxylic acid methyl ester and 110 ml of dimethyl sulfoxide was degassed with nitrogen and heated to ~50° under nitrogen to effect dissolution. The resulting orange solution was treated with 6.0 g (142 mmol) of anhydrous lithium chloride and 7.5 ml of deionized water and heated to 150° under nitrogen, then stirred at 150° for 4 hours. The solution was cooled to 10 to 15°, diluted with 500 ml of 1:1 brine/water and extracted with three 200 ml portions of ethyl acetate. The organic layers were combined and washed with three 200 ml portions of water, two 200 ml portions of brine, dried over anhydrous magnesium sulfate, filtered and concentrated in vacuo to give 28.3 g of the title compound (c), m.p. 39—40°.

NMR ($CDCl_3$, TMS): δ 1.8—2.8 (m, 4H), 3.0—4.3 (m, including 2H broad singlet at 3.53 δ and 3H singlet at 3.80 δ, 6H), 4.8—5.4 (m, 2H), 5.5—6.1 (m, 1H), 6.5—7.4 (m, 3H).

Infrared: $\nu_{max}$ 2922, 1713, 1642, 1599, 1588, 1472, 1441, 1436, 1258, 1081, 910, 771, 719, 609 $cm^{-1}$.

TLC (Silica Gel GF): Rf = 0.32 in 10% ethyl acetate in hexane.

(d) The 2-ethylenedioxy ketal of 1,2,3,4-tetrahydro-5-methoxy-3-(3-propene)naphthalen-2-one

A solution of 27.8 g (128 mmol) of 1,2,3,4-tetrahydro-5-methoxy-3-(3-propene)naphthalen-2-one, 450 ml of methylene chloride, 150 ml (2.2 mol) of ethylene glycol, 60 ml (450 mmol) of triethyl orthoformate, and 270 mg (1.41 mmol) of p-toluenesulfonic acid monohydrate was degassed with nitrogen and stirred at room temperature under nitrogen for 22 hours after which the reaction was quenched with 7.5 ml (52 mmol) of triethylamine, diluted with 500 ml of 1:1 saturated aqueous sodium bicarbonate/water and the layers were separated. The aqueous layer was extracted with 200 ml of methylene chloride. The combined organic layers were washed with three 500 ml portions of water and 500 ml of brine, then concentrated by rotary evaporation to give ~40 g of a red oil. The red oil was dissolved in 200 ml of hexane and treated with 200 ml of water. The mixture was degassed and stirred under nitrogen for one hour. The layers were separated and the organic layer was dried with anhydrous magnesium sulfate, then filtered and concentrated in vacuo to give ~35 g of an orange oil. The orange oil was filtered through 100 g of silica gel 50 washing with 800 ml of 10% ethyl acetate in hexane. The filtrate was concentrated in vacuo to give 31.5 g (94%) of the title compound (d), m.p. 34—35°.

NMR ($CDCl_3$, TMS): δ 1.7—3.3 (m, including 2H broad singlet at 2.90 δ, 7H), 3.4—4.4 (m, including 3H singlet at 3.77 δ, 7H), 4.8—5.3 (m, 2H), 5.6—6.2 (m 1H), 6.5—7.4 (m, 3H).

Infrared: $v_{max}$ (film): 2940, 2890, 1620, 1590, 1470, 1440, 1260, 1155, 1075, 950, 770 $cm^{-1}$.

TLC (Silica Gel GF): Rf = 0.35 in 10% ethyl acetate in hexane.

(e) 2,2-Ethylenedioxy-5-methoxy-1,2,3,4-tetrahydronaphthalen-3-ylacetic acid

To a mixture of 1400 ml of deionized water and 66.5 g (310 mmol) of sodium metaperiodate was added 1.0 g (6.4 mmol) of potassium permanganate. The purple solution was stirred for 30 minutes at room temperature then treated in sequence with 5.0 g (36 mmol) of anhydrous potassium carbonate, then 350 ml of t-butanol, followed by 8.9 g (34 mmol) of the ethylenedioxy ketal of 1,2,3,4-tetrahydro-5-methoxy-3-(3-propene)naphthalen-2-one in 350 ml of t-butanol. The resulting reddish-purple suspension was stirred at room temperature for 2 hours. The reaction was quenched with 10 ml (150 mmol) of ethylene glycol and stirred at room temperature for 2.5 hours. Approximately 30% of the solvent was removed via rotary evaporation, and the remaining material was acidified to pH 3—4 with 100 ml of 1M aqueous hydrochloric acid and extracted with three 500 ml portions of ethyl acetate. The organic layers were combined and washed with two 500 ml portions of brine, dried over anhydrous sodium sulfate, filtered, and the solvents removed in vacuo to give 8.5 g (89%) of the title compound (e), m.p. 129—130°.

NMR ($CDCl_3$, TMS): δ 1.8—3.4 (m, 6H), 3.9—4.5 (m, including 3H singlet at 3.77 δ, 8H), 6.4—7.4 (m, 3H), 10.27 (broad singlet, 1H).

TLC (Silica Gel GF): Rf = 0.20 in 30% ethyl acetate in hexane.

(f) 5-Methoxy-2-oxo-1,2,3,4-tetrahydronaphthalen-3-ylacetic acid

A solution of 8.0 g (28.7 mmol) of 2,2-ethylenedioxy-5-methoxy-1,2,3,4-tetrahydronaphthalen-3-ylacetic acid, 80 ml of 3N aqueous hydrochloric acid, and 80 ml of acetone was degassed and heated to 60° under nitrogen then stirred under nitrogen at 60° for 4 hours. The reaction was cooled to room temperature, approximately 50% of the solvent was removed by rotary evaporation, diluted with 100 ml of brine, and extracted with three 100 ml portions of ethyl acetate. The organic layers were combined and washed with two 100 ml portions of brine, dried over anhydrous sodium sulfate, filtered, and concentrated via rotation evaporation to give an orange solid. The orange solid was triturated with 10 ml of ether and filtered to give 4.9 g (73%) of the title compound (f), m.p. 129—131°.

NMR ($CDCl_3$, TMS): δ 2.2—3.2 (m, 4H), 3.3—4.0 (m, including 2H broad singlet at 3.67 δ and 3H singlet at 3.85 δ, 6H), 6.4—6.9 (m, 2H), 7.1—7.3 (m, 1H), 10.2 (bs, 1H).

Infrared: $v_{max}$ 2908, 2855, 1730, 1714, 1676, 1471, 1454, 1446, 1266, 1202, 1195, 1184, 1091, 776, 747, 724 $cm^{-1}$.

TLC (Silica Gel GF): Rf = 0.22 in 35% ethyl acetate in hexane with 1% acetic acid.

(g) 2,3,3A,4-Tetrahydro-5-methoxy-2-oxo-naphtho[2,3-B]furan

A solution of 5-methoxy-2-oxo-1,2,3,4-tetrahydronaphthalen-3-ylacetic acid (1.75 g, 7.49 mmol) in 88 ml of ethyl acetate was treated all at once with 88 ml of a reagent prepared immediately before use as follows: 20.0 ml of a solution of 0.40 ml of 70% perchloric acid in 100 ml of ethyl acetate was added to 50 ml of ethyl acetate, then 19.2 ml (0.20 mmol) of acetic anhydride was added and the reagent diluted to a total volume of 100 ml with ethyl acetate. The solution was stirred for 10 minutes at room temperature under nitrogen then quenched with 100 ml of saturated aqueous sodium bicarbonate. The layers were separated and the organic layer was washed with 100 ml of brine, dried with anhydrous sodium sulfate, filtered and concentrated in vacuo. To remove the excess acetic anhydride, the red oil was treated with 10 drops of pyridine and 200 ml of methanol. The solvents were removed in vacuo (rotovap bath below 30°); then to remove the pyridine 100 ml of toluene was added and the solvents were removed in vacuo (rotovap bath below 35°). An additional 100 ml of toluene was added and concentrated in vacuo to give a yellow solid.

The yellow solid was recrystallized from ethyl acetate and hexane to give 890 mg (55%) of the title compound (g), m.p. 139—141°.

NMR ($CDCl_3$, TMS): δ 2.0—4.1 (m, including 3H singlet at 3.86 δ, 8H), 6.0—6.2 (d, J=3Hz, 1H), 6.6—7.0 (m, 2H), 7.0—7.4 (m, 1H).

Infrared: $\nu_{max}$ 2926, 1800, 1686, 1571, 1472, 1444, 1267, 1075, 964, 865, 850, 780 $cm^{-1}$.

CMR ($CDCl_3$, TMS): δ ppm (relative intensity): 173.94 (14), 156.31 (17), 154.89 (18), 134.98 (17), 127.79 (92), 121.42 (11), 119.48 (90), 109.60 (97), 101.09 (81), 55.48 (64), 34.76 (88), 33.17 (88), 27.29 (85).

TLC (Silica Gel GF): Rf = 0.32 in 15% ethyl acetate in hexane.

Example 2

15-Cyclohexyl-9,15-dideoxy-13,14-dihydro-2′,9α-methano-3-oxa-4,5,6,16,17,18,19,20-ocatanor-3,7-(1′,3′-interphenylene)$PGF_1$ (Formula I where $R_1$ is H; $L_{20}$ is α-OH,β-H; $M_1$ is H, H; R is cyclohexyl).

(a) 1-Bromo-4-cyclohexylbutane

4-Cyclohexyl-butan-1-ol (8.1 ml, 47 mmol) at −5°C under nitrogen was treated dropwise over 10—15 minutes with 2.2 ml (23 mmol) of phosphorus tribromide, stirred at 0°C for 15 minutes, room temperature for 2 hours, then at 100°C for 1.5 hours before cooling to 0°C. The reaction was quenched with 50 g of ice, diluted with 100 ml of brine, and extracted with ether. The combined organics were washed with brine, dried over anhydrous sodium sulfate, filtered, concentrated, and distilled at 2 mm Hg, 100°C, to give 9.84 g (96%) of 1-bromo-4-cyclohexylbutane as a colorless liquid.

NMR ($CDCl_3$, TMS): δ 0.6—2.3 (m, 17H), 3.43 (t, J=7Hz, 2H).

(b) Dimethylbutyl(4-cyclohexyl)phosphonate

A solution of 6.7 ml (52 mmol) of diethylphosphite in 400 ml of dry tetrahydrofuran was degassed, cooled to −75°C under nitrogen, treated dropwise with 36.4 ml (57 mmol) of 1.57M n-butyllithium in hexane, stirred 30 minutes at −75°C, 30 minutes at 0°C, then treated with 9.4 g (43 mmol) of 1-bromo-4-cyclohexylbutane in 50 ml of tetrahydrofuran dropwise over 10 minutes. The solution was stirred at room temperature for 1 hour, then at 60°C for 4 hours, before cooling to 0°C and quenching with 500 ml of brine containing 40 ml of 1N aqueous hydrochloric acid. The layers were separated and the aqueous layer was extracted with ethyl acetate. The organic layers were combined, washed with brine, dried over anhydrous sodium sulfate, filtered, concentrated and chromatographed on silica gel eluting with ethyl.acetate in hexane to give 9.6 g (81%) of dimethylbutyl(4-cyclohexyl)phosphonate as a colorless oil.

NMR ($CDCl_3$, TMS): δ 0.5—1.9 (m, 25H), 4.13 (two q, 4H).

(c) 15-Cyclohexyl-8,12-dehydro-13,14-dihydro-2′,9α-methano-3-oxa-11-oxo-1,4,5,6,16,17,18,19,20-nonanor-9,11,15-trideoxy-3,7-(1′,3′-interphenylene)-$PGF_1$

A solution of 2.17 g (7.85 mmol) of dimethylbutyl(4-cyclohexyl)-phosphonate in 150 ml of dry tetrahydrofuran was degassed, cooled to −75°C under nitrogen, treated dropwise with 5.0 ml (8.0 mmol) of 1.60 M n-butyllithium in hexane, stirred at −75°C for 1 hour, treated with 0.80 g (3.7 mmol) of 2,3,3A,4-tetrahydro-5-methoxy-2-oxo-naphto[2,3-b]furan (Example 1) in 20 ml of dry tetrahydrofuran, stirred at −75°C for 1 hour, then 2 hours while warming to −40°C, and 15 minutes at ambient temperature. The reaction was cooled to 0°C, treated with 0.21 ml (3.6 mmol) of glacial acetic acid, stirred at room temperature for 15 minutes, then at 55—60°C for 6 hours, cooled to 0°C, and quenched with 250 ml of brine containing 6 ml of 1N aqueous hydrochloric acid. The layers were separated and the aqueous layer was extracted with ethyl acetate. The combined organic layers were washed with 3:1 brine/saturated aqueous sodium bicarbonate, then with brine, dried over anhydrous sodium sulfate, filtered, concentrated *in vacuo*, and chromatographed on silica gel eluting with ethyl acetate in hexane to give 0.855 g (68%) of 15-cyclohexyl-8,12-dehydro-13,14-dihydro-2′,9α-methano-3-oxa-11-oxo-1,4,5,6,16,17,18,19,20-nonanor-9,11,15-trideoxy-3,7-(1′,3′-interphenylene)-$PGF_1$.

NMR ($CDCl_3$, TMS): δ 0.6—3.1 (m, 21H), 3.4—4.1 (m including 3H singled at 3.87, 6H), 6.6—7.3 (m, 3H).

Infrared: (film): 1700, 1655, 1595, 1475, 1410, 1370, 1315, 1270, 1255, 1090, 1080, 765 $cm^{-1}$.

TLC (Silica Gel GF): $R_f$ 0.40 in 20% ethyl acetate in hexane.

(d) 15-Cyclohexyl-13,14-dihydro-2′,9α-methano-3-oxa-11-oxa-1,4,5,6,16,17,18,19,20-nonanor-9,11,15-trideoxy-3,7-(1′,3′-interphenylene)-$PGF_1$

A suspension of 1.37 g (4.05 mmole) of the compound form 2(c), 490 mg of 10% palladium on carbon, and 50 mg (0.36 mmol) of anhydrous potassium carbonate in 75 ml of absolute ethanol was hydrogenated at 3 atm (50 lb/sq in) for 46 hours and then filtered through celite, washing the filter cake with ethyl acetate. The solvents were combined, concentrated in vacuo and chromatographed on silica gel eluting with ethyl acetate in hexane to give 1.10 g (80%) of a 3:1 mixture of the title compound with the corresponding 12-α analog as a colorless oil.

Physical properties for the title compound:

NMR ($CDCl_3$, TMS): δ 0.6—3.3 (m, 26H), 4.83 (s, 3H), 6.6—7.3 (m, 3H).

Infrared: γmax (film): 1740, 1595, 1480, 1450, 1270, 1100, 1055, 975, 885, 780 $cm^{-1}$.

TLC (Silica Gel GF): Rf = 20% ethyl acetate in hexane.

(e) 15-Cyclohexyl-9,15-dideoxy-13,14-dihydro-2',9α-methano-3-oxa-1,4,5,6,16,17,18,19,20-nonanor-3,7-(1',3'-interphenylene)-$PGF_1$

A solution of 400 mg (1.17 mmol) of the mixture of the compound 2(d) with the corresponding 12α-analog was dissolved in 40 ml of absolute ethanol and 4 ml of dry methylene chloride, degassed, cooled to −10°C under nitrogen, treated with 8 ml of ice cold 10% aqueous sodium hydroxide, stirred 15 min, and then treated with 60 mg (1.59 mmol) of sodium borohydride. At 1.0 h, 2.0 h and 6.0 h an additional 60 mg (1.59 mmol) of sodium borohydride was added to the reaction mixture for a total of 240 mg (6.34 mmol) of sodium borohydride. The mixture was stirred for 2.0 hour at −10°C after the last addition, carefully quenched at −10°C with 2.9 ml of glacial acetic acid, diluted with 250 ml of brine, and extracted with ethyl acetate. The organic layers were combined, washed with saturated aqueous sodium bicarbonate and with brine, dried over anhydrous sodium sulfate, filtered, concentrated in vacuo, and chromatographed on Silica Gel eluting with ethyl acetate in hexane to give 373 mg (93%) of the title compound as a white oily solid.

NMR ($CDCl_3$, TMS): δ 0.6—2.9 (m, 28H), 3.83 (s, 3H), 6.6—7.3 (m, 3H).

Infrared: γmax (film): 3350, 1595, 1480, 1455, 1265, 1105, 770, 730 $cm^{-1}$.

TLC (Silica Gel GF): Rf = 0.29 in 20% ethyl acetate in hexane.

(f) 15-Cyclohexyl-9,15-dideoxy-13,14-dihydro-2',9α-methanol-3-oxa-1,2,4,6,16,17,18,19,20-decanor-3,7-(1',3'-interphenylene)-$PGF_1$

A solution of 0.9 ml (5 mmol) of diphenylphosphine in 30 ml of dry tetrahydrofuran under nitrogen at 0°C was treated with 3.2 ml (5.1 mmol) of 1.6 M n-butyllithium in hexane, stirred for 30 minutes at room temperature, then treated with 557 mg (1.6 mmol) of the compound from 2(e) in 10 ml of dry tetrahydrofuran, and heated at 70°C for 7 hours. The orange solution was cooled to 0°C, treated with 0.9 ml (5 mmol) of diphenylphosphine and 3.2 ml (5.1 mmol) of 1.6 M n-butyllithium in hexane, stirred at room temperature for 30 minutes, then at 70°C for 18 hours, before cooling to 0°C, diluting with 100 ml of brine containing 10 ml of 1N aqueous hydrochloric acid, and extracting with ethyl acetate. The combined organics were washed with brine, dried over anhydrous sodium sulfate, filtered, concentrated, and chromatographed on Silica Gel eluting with acetone in methylene chloride to give 509 mg (95%) of the title compound as a white foam.

NMR ($CDCl_3$, TMS): δ 0.5—2.9 (m, 28H), 3.4—3.9 (m, 1H), 6.5—7.2 (m, 3H).

Infrared: γmax (mull): 3430, 3160, 1600, 1465, 1375, 1285, 1260, 1095, 775, 745 $cm^{-1}$.

TLC (Silica Gel GF): Rf = 0.24 in 5% acetone in methylene chloride.

(g) 2-Cyano-5-cyclohexyl-2-decarboxy-9,15-dideoxy-13,14-dihydro-2',9α-methano-3-oxa-4,5,6,16,17,18,19,20-octanor-3,7-(1',3'-interphenylene)-$PGF_1$

A suspension of 450 mg (1.37 mmol) of the compound from 2(f), 1.8 ml (28 mmol) of chloroacetonitrile, and 2.2 g (16 mmol) of anhydrous potassium carbonate in 20 ml of acetone was degassed, heated at 65°C for 24 hours under nitrogen, cooled, diluted with 100 ml of brine, and extracted with ethyl acetate. The combined organics were washed with brine, dried over anhydrous sodium sulfate, filtered, concentrated, and chromatographed on Silica Gel eluting with ethyl acetate in hexane to give 498 (99%) of the title compound as a colorless oil which solidified in the refrigerator.

NMR ($CDCl_3$, TMS): δ 0.6—3.0 (m, 27H), 3.5—3.9 (m, 1H), 4.74 (s, 2H), 6.7—7.3 (m, 3H).

Infrared: γmax (film): 3400, 2350 (w), 1595, 1465, 1450, 1240, 1105, 890, 770 $cm^{-1}$.

TLC (Silica Gel GF): Rf = 0.33 in 30% ethyl acetate in hexane.

(h) 15-Cyclohexyl-9,15-dideoxy-13,14-dihydro-2',9α-methano-3-oxa-4,5,6,16,17,18,19,20-octanor-3,7-(1',3'-interphenylene)-$PGF_1$

A solution of 450 mg (1.22 mmol) of the compound from 2(g) in 30 ml of methanol was treated with 10 ml of 25% aqueous potassium hydroxide, degassed, heated at 90°C under nitrogen for 5 hours, cooled to 0°C, acidified to pH 5—6 with 1N aqueous hydrochloric acid, diluted with 20 ml brine, and extracted with ethyl acetate. The combined organic layers were washed with brine, dried over anhydrous sodium sulfate, filtered, concentrated, and chromatographed on CC-4 acid washed silica gel eluting with ethyl acetate in hexane to give 461 mg (98%) of solid which was crystallized from hot tetrahydrofuran/hexane to give 305 mg of the title compound as a white solid, mp 158—160°C.

NMR (Acetone-$d_6$, TMS): δ 0.6—3.0 (m, 24H), 3.4—4.6 (m, 5H), 4.73 (s, 2H), 6.7—7.3 (m, 3H).

Infrared: γmax (film): 3420, 1735, 1590, 1460, 1235, 1120, 1080, 1015, 785, 720 $cm^{-1}$.

TLC (Silica Gel GF): Rf = 0.57 in the organic phase of 9:2:5:10 ethyl acetate/acetic acid/cyclohexane/water (Rf=0.15 for 6β-$PGI_2$ in the same system).

## Example 3

### Dimethyl[(4R)-4-cyclohexyl-4-tetrahydropyran-2-yloxy-butyl]phosphonate

(a) 1-cyclohexylprop-2-enol (Chart I, Compound 5)

To 140 ml of dry tetrahydrofuran, degassed and flushed with nitrogen (3×) and cooled to 0°C under nitrogen, was added 1.3M vinyl magnesium bromide (195 ml, 253.5 mmol) in tetrahydrofuran rapidly and dropwise over 5 minutes. The resulting solution was stirred for 5 minutes at 0°C under nitrogen after which

a solution of 24.0 g (223 mmol) of cyclohexylcarboxaldehyde in 40 ml of dry tetrahydrofuran was added via syringe at 0°C. The resulting mixture was stirred for 3.75 hours at 0 to 5°C under a nitrogen atmosphere after which the reaction was quenched at 0°C by careful addition of saturated aqueous ammonium chloride. The resulting suspension was poured into 1L of ice cold, saturated, aqueous ammonium chloride and extracted with three 600 ml portions of ethyl acetate. The ethyl acetate extracts were combined and washed with 1L of saturated aqueous ammonium chloride, 1L of saturated aqueous sodium bicarbonate, then twice with 1L each of brine. The ethyl acetate extract was dried thoroughly over magnesium sulfate, filtered, and concentrated at room temperature via rotovap to give 31.0 g of 1-cyclohexylprop-2-enol.

NMR ($CDCl_3$, TMS): δ 0.73—2.67 (m, 12H, $CH_2$, CH), 3.87 (t, 1H, CH—O, J=6 Hz), 6.07—5.43 (m, 2H, CH=), 5.67—6.13 (m, 1H, CH=).

Infrared (film): 3370, 2925, 1450, 1020, 990, 975, 890 $cm^{-1}$.

TLC (Silica Gel GF): $R_f$ = 0.54 in 25% ethyl acetate in hexane.

(b) (R)-1-Cyclohexylprop-2-enol (Chart I, Compound 6)

To 2.2L of methylene chloride, degassed and flushed with nitrogen and cooled to −25°C under nitrogen, was added 72.2 ml of titanium tetraisopropoxide (242.5 mmol) at −25°C under nitrogen. The solution was stirred for 3 to 5 minutes at −25°C after which 62.16 ml of (−)-diisopropyl(D)tartrate (290 mmol) was added at −25°C under nitrogen. A solution of 31.0 g (214 mmol) of 1-cyclohexylprop-2-enol in 50 ml of methylene chloride was added to the reaction mixture at −25°C under nitrogen. The resulting solution was stirred for 5—10 minutes at −25°C under nitrogen after which 3M t-butylhydroperoxide in dichloroethane (48.5 ml, 145.5 mmol) was added at −25°C under nitrogen. The mixture was stirred for 10 minutes at −25 to −20°C under nitrogen, then stirred for 3 days at −20°C. The reaction was quenched by cannulating the reaction mixture (at −20°C) into a mechanically stirred tartaric acid-ferrous sulfate solution (200 g/400 g in 2L water) at 0°C. The resulting suspension was stirred at 0°C for 20 to 30 minutes and filtered through a pad of celite, washing the pad thoroughly with methylene chloride. The filtrate layers were separated and the aqueous layer was extracted with methylene chloride (2×500 ml each), dried over magnesium sulfate, filtered and concentrated at room temperature via rotovap to give the title compound 10(b) as a yellow oil which was purified as follows: The oil was dissolved in 650 ml of hexane and cooled to 0°C under nitrogen then treated with aqueous 1N sodium hydroxide (550 ml) at 0°C under nitrogen. The resulting suspension was stirred for 40 minutes at 0°C under nitrogen after which the layers were separated and the aqueous layer was extracted with hexane (2×500 ml each). The organic extracts were combined, washed with brine (500 ml), dried over sodium sulfate, filtered and concentrated at room temperature via rotovap to a yellow oil. The yellow oil was chromatographed on silica gel (1200 g) packed with 10% ethyl acetate in Skellysolve B (SSB) eluting with 12% in SSB to give 9.59 g of the title compound 10(b).

NMR ($CDCl_3$, TMS): δ 0.73—2.67 (m, 12H, $CH_2$, CH), 3.87 (e, 1H, CH—O), 5.07—5.43 (m, 2H, CH=), 5.67—6.13 (m, 1H, CH=).

Infrared (film): 3370, 2925, 1450, 1020, 990, 975, 890 $cm^{-1}$.

TLC (Silica Gel GF): $R_f$ = 0.54 in 25% ethyl acetate in hexane.

(c) 3-Cyclohexyl-3-tetrahydropyranyloxy-prop-1-ene (Chart I, Compound 7)

A solution of 22.07 g of (R)-1-cyclohexylprop-2-enol in 300 ml of methylene chloride, degassed and washed with nitrogen, was treated at ambient temperature under nitrogen with pyridinehydrochloride (0.145 g) and then with dihydropyran (44.4 ml, 466 mmol). The reaction mixture was stirred overnight at ambient temperature under nitrogen, then cooled using an ice bath and treated with aqueous sodium bicarbonate (15 ml). The resulting solution was diluted with saturated aqueous sodium bicarbonate (200 ml), stirred for 5 minutes after which the layers were permitted to separate. The organic layer was washed with 200 ml of brine, dried over anhydrous sodium sulfate, filtered and the filtrate concentrated via rotovap to give 35.0 g of compound 10(c) as a yellow oil.

NMR ($CDCl_3$, TMS): δ 0.63—2.20 (m, 17H, $CH_2$, CH), 3.27—4.10 (m, 3H, CH—O, $CH_2O$), 4.67 (bs, 1H, CH—O, THP), 4.93—5.33 (m, 2H, CH=), 5.40—6.13 (m, 1H, CH=).

Infrared (film): 2925, 2855, 1130, 1115, 1080, 1035, 1020, 1015, 995, 980 $cm^{-1}$.

TLC (Silica Gel GF): $R_f$ = 0.62 in 25% ethyl acetate in hexane.

(d) 3-Cyclohexyl-3-tetrahydropyranyloxypropanol
(Chart I, Compound 8)

A solution of 35.0 g of 3-cyclohexyl-3-tetrahydropyranolprop-1-ene (157 mmol) in 795 ml of dry tetrahydrofuran, degassed and flushed with nitrogen, then cooled to 0°C, was treated dropwise at 0°C with 0.5M 9-BBN in tetrahydrofuran (795 ml, 398 mmol). The resulting solution was stirred for one hour at 0°C after which the cooling bath was removed and stirring was continued at ambient temperature for 6 hours. The reaction mixture was then cooled to 0°C and treated slowly with 30% hydrogen peroxide (231 ml), then treated with 3N potassium hydroxide (231 ml) all at once. The resulting suspension was stirred for 35 minutes at 0°C after which the cooling bath was removed and the reaction suspension was stirred for one hour at ambient temperature. The reaction mixture was then diluted with brine (1L). the layers separated, and the aqueous layer was extracted with three 700 ml portions of ethyl acetate. The organic layers were combined and washed with three 500 ml portions of brine, dried over anhydrous sodium sulfate, filtered

and the filtrate concentrated in vacuo at ~25°C. The resulting product was chromatographed on slica gel (1750 g) packed with 5% ethyl acetate in SSB, eluting with 8L of 5% ethyl acetate in SSB, 6L of 10% ethyl acetate in SSB, 6L of 20% ethyl acetate in SSB, 6L of 25% ethyl acetate in SSB, an 4L of 30% ethyl acetate in SSB to give 27.19 g of compound 10(d).

NMR ($CDCl_3$, TMS): δ 0.63—2.90 (m, 20H, $CH_2$, CH, C—OH), 3.23—4.13 (m, 5H, CH—O, $CH_2$—O), 4.03—4.87 (m, 1H, CH—O, THP).

Infrared (film): 3435, 2930, 2855, 1450, 1160, 1135, 1075, 1025, 990 $cm^{-1}$.

TLC (Silica Gel GF): $R_f$ = 0.21—0.38 in 30% ethyl acetate in hexane.

(e) The 1-p-toluenesulfonyl derivative of 3-cyclohexyl-3-tetrahydropyranyloxypropanol

A solution of 27.19 g (112 mmol) of 3-cyclohexyl-3-tetrahydropyranyloxypropanol in 136 ml of dry pyridine, degassed with nitrogen and cooled to 0°C, was treated with 25.7 g (135 mmol) of p-toluenesulfonyl chloride. The reaction mixture was stirred for 20 hours at 0°C under nitrogen after which 350 g of ice was added and the cooling bath was removed. The reaction mixture was stirred for 75 minutes, then diluted with 600 ml of water, and extracted with three 500 ml portions of ethyl acetate. The organic layers were combined, washed with 600 ml of saturated aqueous sodium bicarbonate, 600 ml of water, and 600 ml of brine, and dried over anhydrous sodium sulfate, filtered and the filtrate concentrated via rotovap at room temperature. The residual pyridine was removed azeotropically at room temperature via rotovap using two 300 ml portions of toluene to give 38.09 g of compound 10(e).

NMR ($CDCl_3$, TMS): δ 0.63—2.20 (m, 19H, $CH_2$, CH), 2.47 (s, 3H, $ArCH_3$), 3.23—4.40 (m, 5H, CH—O, $CH_2$—O), 4.47 (m, 1, CH—O, THP), 7.37 (D, 2H, ArH; J=10, 5Hz), 7.87 (D, 2H, ArH; J=10, 5Hz).

Infrared (film): 2930, 2860, 1600, 1445, 1375, 1175, 905, 815, 670 $cm^{-1}$.

TLC (Silica Gel GF): $R_f$ = 0.48 in 20% ethyl acetate in hexane.

(f) (1-Tetrahydropyranyloxy-3-iodopropyl)cyclohexane

(Chart I, Compound 11)

A solution of 36.74 g (92.65 mmol) of the compound from Example (e), 1.5 ml of diisopropylethylamine, 360 ml of acetone and 83.33 g (550 mmol) of sodium iodide was stirred at room temperature under nitorogen for 20 hours. The solution was then cooled using an ice bath and concentrated via rotovap at room temperature to give a red-orange solid. The solid was dissolved in 1L of ethyl of ethyl acetate. The organic layers were washed with 525 ml of 5% aqueous sodium thiosulfate then with 1L of brine, dried over anhydrous magnesium sulfate, filtered and the filtrate concentrated via rotovap at room temperature to give a yellow oil. The oil was chromatographed on 900 g of silica gel packed with SSB, eluting with 4L of SSB, then with 3% ethyl acetate in SSB to give 27.47 g of compound 10(f).

NMR ($CDCl_3$, TMS): δ 0.63—2.53 (m, 19H, $CH_2$, CH), 3.07—3.70 (m, 4H, $CH_2$—O, $CH_2$—I), 3.77—4.10 (m, 1H, CH—O), 4.48—4.82 (m, 1H, CH—O, THP).

Infrared (film): 2925, 2850, 1450, 1200, 1130, 1115, 1075, 1065, 1035, 1023, 980 $cm^{-1}$.

TLC (Silica Gel GF): $R_f$ = 0.47 in 10% ethyl acetate in hexane.

(g) [(4R)-4-Cyclohexyl-4-tetrahydropyran-2-yloxybutyl]phosphonate (Chart I, Compound 12)

To 500 ml of dry tetrahydrofuran cooled to −40°C under nitrogen was added 9.98 ml (96.7 mmol) of diethylamine. The solution was treated with 60 ml (96.7 mmol) of n-butyllithium (1.55M in hexane) dropwise maintaining the temperature below −30°C. The solution was stirred at −35° to −30°C for 15 minutes then cooled to −75°C. A solution of 10.6 g (85.4 mmol) of dimethylmethyl phosphonate in 50 ml of dry tetrahydrofuran was added dropwise maintaining the temperature below −70°C. The solution was stirred for 30 minutes at −75°C to 70°C after which 27.29 g (77.5 mmol) of (1-tetrahydropyranyloxy-3-iodopropyl)cyclohexane in 100 ml of dry tetrahydrofuran was added dropwise maintaining the temperature below −70°C. The reaction mixture was stirred at −70°C for one hour then allowed to warm to −10°C over 4 hours. The reaction was quenched with 800 ml of 1:1 brine/water and the layers separated. The aqueous layer was extracted with two 650 ml portions of ethyl acetate. The organic layers were combined, washed with 800 ml of brine, dried over anhydrous sodium sulfate, filtered and the filtrate concentrated in vacuo. The resulting product was chromatographed on 500 g of silica gel packed with ethyl acetate eluting the product with 2L of ethyl acetate and then with 6L of 5% acetone in ethyl acetate o give 18.14 g of compound 10(g).

NMR ($CDCl_3$, TMS): δ 0.63—2.53 (m, 23H), 3.23—4.20 (m, 3H), 3.70 (s, 3H), 3.83 (s, 3H), 4.60 (bs, 1H).

Infrared (film): 2930, 3850, 1450, 1245, 1200, 1130, 1115, 1060, 1030, 1030, 990, 835, 815 $cm^{-1}$.

TLC (Silica Gel GF): $R_f$ = 0.14 in ethyl acetate.

## Example 4

15-Cyclohexyl-9,11-dideoxy-13,14-dihydro-2',9α-methano-11α-methyl-3-oxa-4,5,6,16,17,18,19,20-octanor-3,7-(1',3'-interphenylene)-$PGF_1$ (Formula I where $R_1$ is H, $L_{20}$ is α-$CH_3$, β-H, $M_1$ is α-OH, β-H and R is cyclohexyl).

(a) 15-Cyclohexyl-8,12-didehydro-9,11-dideoxy-13,14-dihydro-2',9a-methano-3-oxoa-11-oxo-1,4,5,6,16,17,18,19,20-nonanor-3,7-(1',3'-interphenylene)-$PGF_1$, 15-(tetrahydropyranyl ether)

A solution of 11.9- g (35.37) mmol of the product of Example 3 and 450 ml of dry tetrahydrofuran, degassed and flushed with nitrogen, was cooled to −78°C. The stirred solution was treated with 22.5 ml

(36.0 mmol) of 1.60M n-butyllithium dropwise over 15 to 20 minutes, then stirred one hour at −78°C. A solution of 3.71 g (17.17 mmol) of 2,3,3A,4-tetrahydro-5-methoxy-2-oxo-naphtho[2,3-b]furan (Example 1) in 70 ml of dry tetrahydrofuran, degassed and flushed with nitrogen and cooled to −78°C under nitrogen, was added via cannula and under nitrogen pressure dropwise over 30 minutes. The resulting solution was stirred for 4 hours while allowing the temperature to rise slowly to −10°C after which the solution was treated dropwise with 1.03 ml (18 mmol) of glacial acetic acid. The reaction mixture was stirred for 15 minutes at ambient temperature and heated at 60 to 65° for 6 hours. The resulting yellow-green solution was cooled to 5°, neutralized to about pH 6 to 7 with 500 ml of brine containing 18 ml (18 mmol) of 1M aqueous hydrochloric acid, and extracted with three 250 ml portions of ethyl acetate. The organic layers were combined and washed with 200 ml of 3:1 brine/saturated aqueous sodium bicarbonate and then with 400 ml of brine and dried over anhyydrous sodium sulfate, filtered and concentrated in vacuo. The resulting crude product was chromatographed on 200 g of silica gel to give the title compound 4(a).

(b) 15-Cyclohexyl-9,11-dideoxy-13,14-dihydro-2′,9a-methano-3-oxa-11-oxo-1,4,5,6,16,17,18,19,20-nonanor-3,7-(1′,3′-interphenylene)-12-epi-$PGF_1$, 15-(tetrahydropyranyl ether)

To a solution of 4.95 g of the compound of Example 4 (a) in 250 ml of degassed ethanol was added a solution of 1.67 g (1.56 g/atom) of 10% palladium on carbon and 112 mg (0.81 mmol) of anhydrous potassium carbonate. The resulting mixture was hydrogenated at 345 kPa (50 psi, 93.4 atm) for 42 hours after which the mixture was filtered through a pad of 1:1 celite/anhydrous magnesium sulfate (30 g). The filter cake was washed with two 200 ml portions of ethyl acetate. The colorless solution was concentrated in vacuo using 200 ml of toluene to azeotrope the last traces of water and ethanol to give 5.2 g of colorless oil which was filtered through 20 g of silica gel 60 washing with 500 ml of 20% ethyl acetate in hexane to give compound 4(b).

(c) 15-Cyclohexyl-9,11-dideoxy-13,14-dihydro-2′,9a-methano-3-oxa-11-oxo-1,4,5,6,16,17,18,19,20-nonanor-3,7-(1′,3′-interphenylene)-$PGF_1$, 15-(tetrahydropyranyl ether)

To 4.7 g of the compound of Example 4(b) in 450 ml of 95% ethanol was added 90 ml of 10% aqueous sodium hydroxide and the resulting solution was degassed and flushed with nitrogen and heated at reflux (bath temperature 105°) for 7.5 hours under nitrogen. The reaction was cooled to room temperature and approximately two-thirds of the solvent was removed in vacuo at room temperature and the remaining material was diluted with 500 ml of brine and extracted with three 200 ml portions of ethyl acetate. The combined organics were washed with 200 ml of brine, dried over anhydrous sodium sulfate, filtered and concentrated in vacuo. The crude product was flash chromatographed on silica gel to give the title compound 4(c).

(d) (15R)-15-Cyclohexyl-9,11-dideoxy-13,14-dihydro-2′,9α-methano-11-methylene-1,4,5,6,16,17,18,19,20-nonanor-3-oxa-3,7-(1′,3′-interphenylene)-$PGF_1$, 15-(tetrahydropyranylether)

A degassed solution of methyl phenyl-N-methyl sulfoximine (1.502 g, 8.88 mmol) in freshly distilled tetrahydrofuran (26.6 ml) was cooled to −78°C under nitrogen and treated dropwise with 2.9M methylmagnesium chlorie in tetrahydrofuran (3.06 ml, 8.88 mmol). The resulting solution was stirred for 35 minutes at −78°C and for 15 minutes at 0°C, cooled to −78°C and treated with a solution of the product 4(c) (1.92 g. 4.36 mmol) in freshly distilled tetrahydrofuran (10 ml). Residual ketone starting material was transferred to the reaction with two 4 ml aliquots of freshly distilled tetrahydrofuran. The reaction was stirred for 1.75 hours while the temperature was permitted to from −78°C to 0°C, then was stirred for 2 hours at 0°C. The reaction was diluted with ice-cold brine (80 ml) and extracted with diethyl ether (3 × 110 ml). The ether extracts were washed with brine (80 ml), 0.2 M aqueous potassium bisulfate (80 ml), aqueous saturated sodium bicarbonate (80 ml) and brine (80 ml), dried over magnesium sulfate, filtered and concentrated in vacuo to a yellow oil (3.49 g).

A degassed solution of the crude sulfoximine (3.37 g; theory 2.65 g) in freshly distilled tetrahydrofuran (66) was cooled to 0°C under nitrogen and treated with 50% acetic acid/water (20 ml), followed immediately by aluminum amalgam which had been prepared by washing 20 mesh aluminum powder (3.55 g) with ether (75.5 ml) methanol (2 × 75.5 ml) then 3.57 g of mercuric chloride in water (122 ml) followed by methanol (75.5 ml) and ether (75.5 ml).

The resulting black suspension was permitted to stir for 2.75 hours while the reaction temperature was allowed to go slowly from 0°C to 10°C, cooled to 0°C, diluted with ethyl acetate (100 ml) and stirred for 30 minutes at 0°C. The suspension was filtered through celite, and the filtercake was washed with ethyl acetate. The combined filtrate was washed with brine (135 ml) 0.2M aqueous potassium bisulfite (135 ml) and brine (135 ml), dried over sodium sulfate, filtered and concentrated to a yellow oil.

The crude product was chromatographed on silica gel in 5% ethyl acetate in hexane to give the title compound 11(d).

NMR ($CDCl_3$, TMS): δ 0.73—3.10 (m, 30), 3.27—3.63 (m, 2), 3.77—4.23 (m, l), 3.80 (S, 3), 4.53—4.73 (m), 4.77—4.97 (m, 2), 6.63—6.90 (m, 2), 7.13 (d of d, l, $J_1$=$J_2$=7.5Hz).

Infrared (film): 2930, 2860, 1652, 1605, 1595, 1475, 1455, 1405, 1260, 1240, 1205, 1135, 1115, 1095, 1080, 1028, 995, 865, 768 $cm^{-1}$.

TLC (Silica Gel GF): $R_f$ = 0.58 in 1% ethyl acetate in hexane.

(e) 15-Cyclohexyl-9,11-dideoxy-13,14-dihydro-2′,9α-methano-11-methyl-1,4,5,6,16,17,18,19,20-nonanor-3-oxa-3,7-(interphenylene)-$PGF_1$, 15-tetrahydropyranyl ether

A degassed solution of the product of 4(d) (0.156 g, 0.36 mmol) in absolute ethanol (11.15 ml) was

treated at room temperature under nitrogen with 10% palladium on charcoal (0.052 g) and anhydrous potassium carbonate (0.06 g). The resulting suspension was alternately degassed and flushed with nitrogen then degassed and flushed with hydrogen and hydrogenated at 345 kPa (50 p.s.i.) for 22 hours. The suspension was evaporated and flushed with nitrogen, filtered through 1:1 celite/magnesium sulfate (3g). The filtercake was washed with ethyl acetate and the combined filtrate was concentrated in vacuo to give 0.155 g of the title compound (e) as a crude oil.

NMR ($CDCl_3$, TMS): δ 0.70—3.21 (m, including doublet, 3, $CH_3$ at 0.90, J=6Hz), 3.27—3.70 (m, 2), 3.80—4.30 (m, l), 3.83, (s, 3), 4.60—4.90 (m, l), 6.70—7.03 (m, 2), 7.13 (d or d, l, $J_1$=$J_2$=7.5Hz).

TLC (Silica Gel GF): $R_f$ = 0.58 in 10% ethyl acetate/hexane.

(f) 15-Cyclohexyl-9,11-dideoxy-13,14-dihydro-2',9α-methano-11-methyl-1,4,5,6,16,17,18,19,20-nonanor-3-oxa-3,7-(1',3'-interphenylene)-$PGF_1$

The crude product of example 4(e) in 8 ml of 4:2:2 acetic accid/water/tetrahydrofuran was stirred at 45°C under nitrogen for 3 hours, cooled, diluted with brine and extracted with ethyl acetate. The organics were washed with brine, dried over sodium sulfate, filtered and concentrated to a yellow oil.

The crude product was chromatographed on silica gel in 10% ethyl acetate in hexane to give 0.116 g (90%) of the title compound (f).

NMR ($CDCl_3$, TMS): δ 0.70—3.03 (m, 29, including doublet, 3, J=6Hz), 3.20—3.53 (m, l), 3.83 (s, 3), 6.63—6.97 (m, 2), 7.13 (d of d, l, $J_1$=$J_2$=7.5Hz).

Infrared (film): 3370, 2930, 2850, 1605, 1595, 1475, 1444, 1375, 1330, 1310, 1260, 1100, 1080, 1045, 970, 895, 775, 735 $cm^{-1}$.

TLC (Silica Gel GF): $R_f$ = 0.51 in 25% ethyl acetate in hexane.

(g) 15-Cyclohexyl-1,2,4,5,6,16,17,18,19,20-decanor-9,11-dideoxy-13,14-dihydro-2',9α-methano-11-methyl-3-oxa-3,7-(1',3'-interphenylene)-$PGF_1$

A degassed solution of diphenylphosphine (0.173 ml, 0.973 mmol), in freshly distilled tetrahydrofuran (5.5 ml) was cooled to 0°C under nitrogen and treated with 1.58 M n-butyllithium (0.60 ml, 0.95 mmol). The resulting red solution was stirred for 5 minutes at 0°C and for 30 minutes at room temperature then treated at ambient temperature with a solution of the product of 4(f) (0.116 g, 0.325 mmol) in freshly distilled tetrahydrofuran (1.1 ml) Residual compound was transferred to the reaction vessel with two 0.27 ml aliquots of freshly distilled tetrahydrofuran, and the reaction was stirred at reflux for 6 hours, cooled to 0°C, treated with diphenylphosphine (0.52 ml, 2.92 mmol) followed by n-butyllithium (1.8 ml, 2.85 mmol). The reaction was stirred for 5 minutes at 0°C, 20 min at ambient temperature and 18 hours at reflux, cooled to 0°C, acidified with cold, aqueous 1*N* HCl (12 ml) and diluted with ice-cold brine. The resulting suspension was extracted with ethyl acetate and the organics were washed with brine, dried over sodium sulfate, filtered and concentrated to a semi-solid.

The crude product was chromatographed on silica gel in 15% ethyl acetate in hexane to give 0.106 g (95%) of the title compound (g).

NMR ($CDCl_3$, TMS): δ 0.70—3.00 (m, 29, including doublet, 3 at 0.90, J=6Hz), 3.23—3.57 (m, l), 5.33—6.43 (m, l), 6.63—6.90 (m, 2), 7.07 (d of d, l).

Infrared (film): 3350, 2930, 2850, 1605, 1595, 1470, 1455, 1380, 1290, 1245, 1085, 1050, 1000, 895, 775, 740 $cm^{-1}$.

TLC (Silica Gel GF): Rf = 0.31 in 25% ethyl acetate in hexane.

(h) 2-Cyano-15-cyclohexyl-2-decarboxy-9,11-dideoxy-13,14-dideoxy-2',9α-methano-11-methyl-1,4,5,6,17,18,19,20-nonanor-3-oxa-3,7-(1',3'-interphenylene)-$PGF_1$ and its 8,9,11,12-tetra-epi isomer

A degassed solution of the product of 4(g) (0.106 g, 0.309 mmol) in acetone (5 ml) was treated at ambient temperature under nitrogen with anhydrous potassium carbonate (0.915 g, 6.62 mmol), followed by chloroacetonitrile (0.71 ml, 11.25 mmol). The resulting suspension was stirred at reflux for 22 hours and was incomplete. Additional potassium carbonate (0.915 g, 6.62 mmol) and chloroacetonitrile (0.71 ml, 11.25 mmol) was added, and the reaction was stirred at reflux for 24 hours, cooled and diluted with 1:1 brine/water (75 ml). The suspension was extracted with ethyl acetate (3 × 75 ml), and the combined extracts with ethyl acetate (3 × 75 ml), and the combined extracts were washed with brine (2 × 75 ml), dried over sodium sulfate, filtered and concentrated to a brown oil.

The crude product was chromatographed on silica gel acetone in methylene chloride to give 0.077 g (65%) of title compound (h).

NMR ($CDCl_3$, TMS): δ 0.70—3.03 (m, 29, including doublet, 3, J=6Hz at 0.90), 3.20—3.53 (m, l), 4.77 (s, 2), 6.73—7.03 (m, 2), 7.17 (d of d, $J_1$=$J_2$=7.5Hz).

Infrared (film): 3400, 2930, 2850, 1605, 1590, 1480, 1475, 1375, 1260, 1235, 1100, 1045, 980, 895, 775, and 740 $cm^{-1}$.

TLC (Silica Gel GF): $R_f$ = 0.80 in 5% acetone in methylene chloride.

(i) 15-Cyclohexyl-9,11-dideoxy-13,14-dihydro-2',9α-methano-11-methyl-4,5,6,16,17,18,19,20-octanor-3-oxa-3,7-(1',3'-interphenylene)-$PGF_1$

A degassed solution of the nitrile compound 4(h) (0.077 g, 0.202 mmol) in anhydrous methanol (4.56 ml) was treated at ambient temperature under nitrogen with 25% aqueous potassium hydroxide (1.4 ml). The resulting solution was stirred at reflux for 5.5 hours, cooled to 0°C, acidified with aqueous 1N HCl (10

ml) and diluted with ice-cold brine (40 ml). The resulting suspension was extracted with ethyl acetate (3 × 50 ml), and the combined extracts were washed with brine (2 × 50 ml), dried over sodium sulfate, filtered and concentrated to an off-white solid which was recrystallized from ethyl acetate/hexane to give 0.056 g, (69%) of title compound (i), m.p. 123—125°C.

NMR ($CDCl_3$, TMS): δ 0.70—3.10 (m, 28, including doublet, 3, J=6Hz at 0.90), 3.20—3.53 (m, I), 4.30 (bs, 2), 4.63 (s), 6.45—7.45 (m, 3).

Infrared (mull): 3430, 2970, 2860, 2720, 2580, 1740, SH(1705), 1605, 1590, 1465, 1425, 1380, 1260.

TLC (Silica Gel GF): $R_f$ = 0.32 in 1:1 A-IX-cyclohexane.

Example 5

(a) 15-Cyclohexyl-9,11-dideoxy-13,14-dihydro-2′,9α-methano-11-methylene-1,4,5,6,16,17,18,19,20-nonanor-3-oxa-3,7-(1′,3′-interphenylene)-$PGF_1$

A solution of example 4(a) (0.195 g, 0.44 mmol) in acetic acid (6 ml) water (3 ml) and tetrahydrofuran (1.5 ml) was stirred for 3 hours at 45°C under nitrogen, cooled diluted with brine (75 ml) and extracted with ethyl acetate (3 × 75 ml). The organics were washed with brine (75 ml), aqueous saturated sodium bicarbonate (3 × 75 ml), and brine (2 × 75 ml), dried over sodium sulfate, filtered and the filtrate concentrated in vacuo to give a pale yellow oil.

The crude product was chromatographed on 10% ethyl acetate in hexane to afford 0.109 g (70%) of title compound (a).

NMR ($CDCl_3$, TMS): δ 0.73—3.10 (m, 24), 3.20—3.63 (m, I, 3.83 (s, 3), 4.77—5.07 (m, 2), 6.73—6.97 (m, 2), 7.13, (d of d, I, $J_1$=$J_2$=7.5Hz), 7.27—7.80 (m, I).

Infrared (film): 3400, 3330, 2930, 2860, 1660, 1605, 1595, 1475, 1450, 1335, 1330, 1270, 1255, 1130, 1100, 1070, 1035, 965, 895, 875, 775, 754 $cm^{-1}$.

TLC (Silica Gel GF): $R_f$ = 0.47 in 20% ethyl acetate in hexane.

(b) 15-Cyclohexyl-1,2,4,5,6,16,17,18,19,20-decanor-9,11-dideoxy-13,14-dihydro-2′,9α-methano-11-methylene-3-oxa-3,7-(1′,3′-interphenylene)-$PGF_1$

A degassed solution of diphenylphosphine (0.16 ml, 0.90 mmol) in freshly distilled tetrahydrofuran (5 ml) was cooled to 0°C under nitrogen and treated with 1.58 M n-butyllithium in hexane (0.55 ml, 0.87 mmol). The resulting red solution was stirred at 0°C for 5 minutes and at ambient temperature for 3 minutes then treated at room temperature with a solution of the methyl ester from 5(a) (0.106 g, 0.30 mml) in freshly distilled tetrahydrofuran (1 ml). The reaction was stirred at reflux for 6 hours, cooled to 0°C, treated with diphenylphosphine (0.32 ml, 1.80 mmol) followed by 1.58 M n-butyllithium in hexane (1.10 ml, 1.74 mmol). The reaction was stirred at 0°C for 5 minutes, at ambient temperature for 15 minutes and at reflux for 18 hours. The reaction was cooled to 0°C, diluted with brine (40 ml) containing 5 ml of 1N HCl, and extracted with ethyl acetate (3 × 35 ml). The organics were washed with brine (3 × 50 ml), dried over sodium sulfate, filtered and concentrated to a semi-solid.

The crude product was chromatographed on silica gel with 15% ethyl acetate in hexane to give 0.065 g (64%) of title product (b).

NMR ($CDCl_3$, TMS): δ 0.73—3.03 (m, 25), 3.27—3.6- (m, I), 4.87 (d, 2, J=7Hz), 5.10—5.97 (bs, I), 6.70 (2d, 2, J1=J2=7.5Hz).

Infrared (film): 3340, 2930, 2850, 1710 (weak), 1655, 1590, 1465, 1455, 1445, 1330, 1285, 1060, 1040, 880, 775, 735 $cm^{-1}$.

TLC (Silica Gel GF): Rf = 0.29 in 25% in ethyl acetate in hexane.

(c) 2-Cyano-15-cyclohexyl-9,11-dideoxy-13,14-dihydro-2′,9α-methano-1l-methylene-1,4,5,6,16,17,18,19,20-nonanor-3-oxa-3,7-(1′,3′-interphenylene)-$PGF_1$

A degassed solution of the phenol compound 5(b) (0.065 g, 0.19 mmol) in acetone (3 ml) was treated at ambient temperature under nitrogen with anhydrous potassium carbonate (0.566 g, 4.09 mmol) followed by chloroacetonitrile (0.44 ml, 6.97 mmol). The resulting suspension was stirred at reflux for 29.5 hours at reflux, cooled and diluted with 1:1 brine/water (50 ml). The suspension was extracted with ethyl acetate (3 × 50 ml), and the organics were washed with brine (2 × 50 ml), dried over sodium sulfate, filtered and concentrated to a brown oil.

The crude product was chromatographed on silica gel with ethyl acetate in hexane to give 0.067 g (93%) of title compound (c).

NMR ($CDCl_3$, TMS): δ 0.73—3.10 (m, 25), 3.23—3.63 (m, I), 4.80 (s, 2), 4.87 (d, 2, J=7HZ), 6.87 (2d, 2, $J_1$=$J_2$=7.5Hz), 7.20 (d of d, 1, $J_1$=$J_2$7.5Hz).

Infrared (film): 3400, 3065, 2930, 2860, 1645, 1590, 1475, 1450, 1265, 1235, 1100, 885, 765 $cm^{-1}$.

TLC (Silica Gel GF): Rf = −.54 in 5% acetone in methylene chloride.

(d) 15-Cyclohexyl-9,11-dideoxy-13,14-dihydro-2′,9α-methano-11-methylene-4,5,6,16,17,18,19,20-octanor-3-oxa-3,7-(1′,3′-interphenylene)-$PGF_1$

A degassed solution fo the nitrile compound 5(c) (0.067 g, 0.177 mmol) in absolute methanol (4 ml) was treated at ambient temperature under nitrogen with 25% aqueous potassium hydroxide (1.4 ml). The resulting solution was stirred at reflux for 6 hours, cooled to 0°C, acidified to ~pH 4 with 1N aqueous HCl (9.5 ml) and diluted with brine 35 (35 ml). The aqueous suspension was extracted with ethyl acetate (3 × 35

ml), and the combined organics were washed with brine (2 × 45 ml), dried over sodium sulfate, filtered and concentrated to a light yellow solid.

The crude product was recrystallized from ethyl acetate-hexane ~1:10 to give a total of 60 mg (85%) of title compound (d).

NMR ($CDCl_3$, TMS): δ 0.73—3.10 (m, 24), 3.20—3.80 (m, 3), 4.63 (s, 2), 4.80 (d, 2, J=7HZ, 6.58 (d, I, J=7.5Hz), 6.78 (d, I, J=7.5Hz), 7.05 (d of d, I, $J_1$=$J_2$=7.5Hz).

Infrared (nujol mull): 3350, 2930, 2860, 2550, 2430, 1715, 1605, 1590, 1470, 1440, 1375, 1335, 1255, 1235, 1115, 1040, 875 and 770 $cm^{-1}$.

TLC (Silica Gel GF): Rf = 0.43 in 2 A-IX-I cyclohexane, Rf = 0.31 in 1:1-A-IX-cyclohexane.

FORMULA CHART

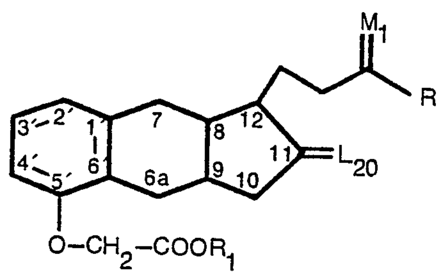

Formula I

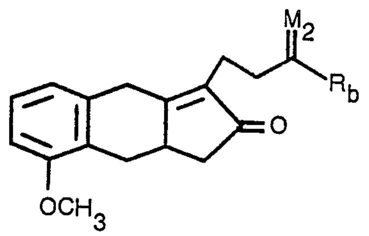

Formula I (d)

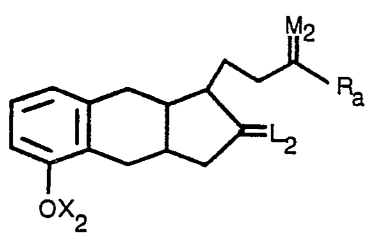

Formula I (e)

CHART A

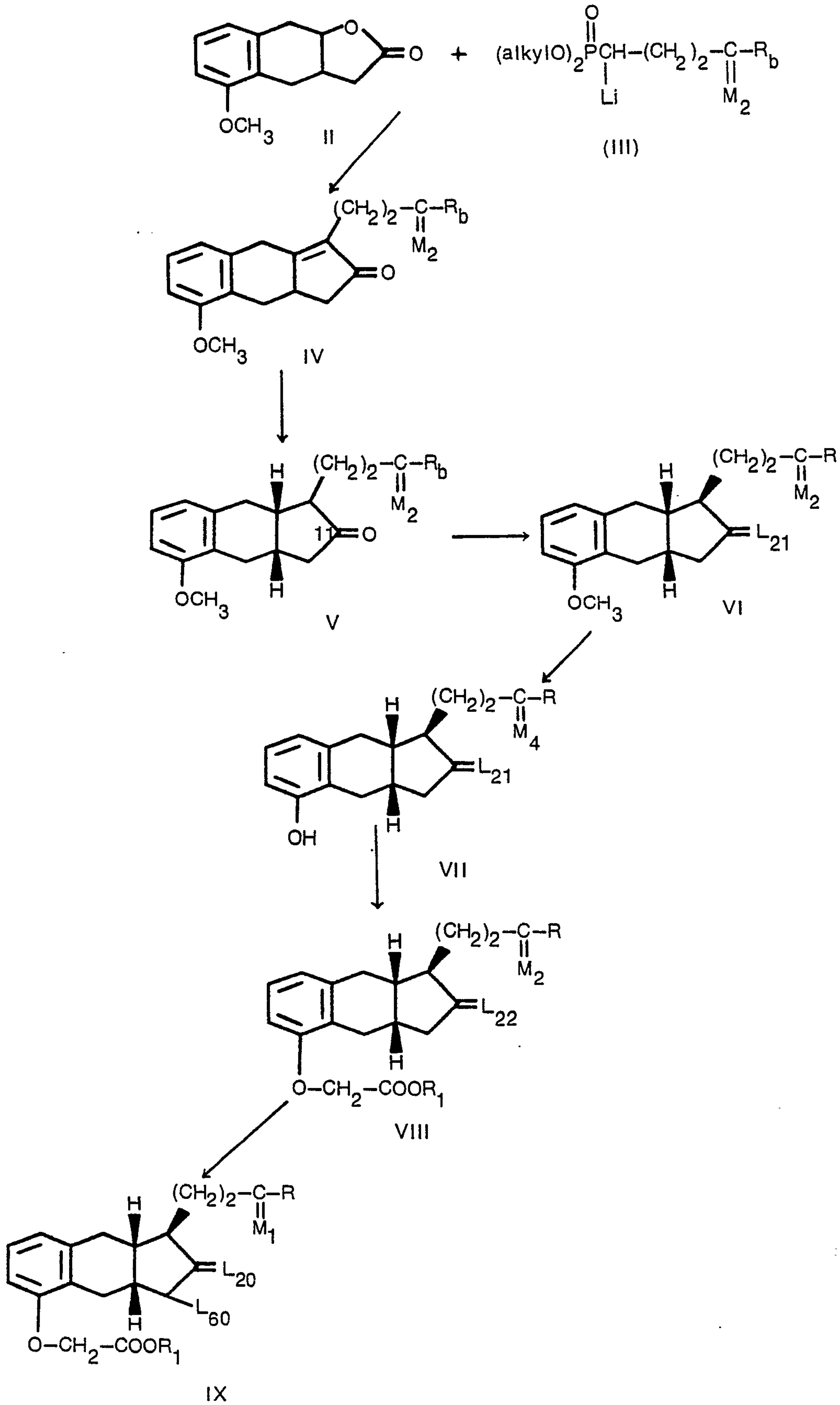

II

(III)

IV

V

VI

VII

VIII

IX

CHART B

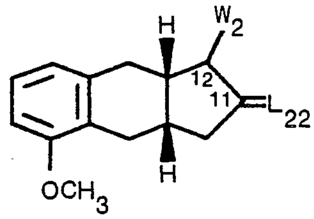

+

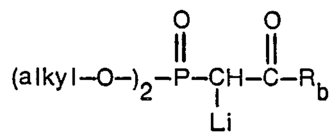

Formula X

| Formula | $W_2$ |
| --- | --- |
| XI | CHO |
| XII | 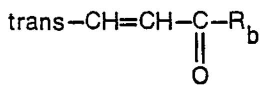 |
| XIII | 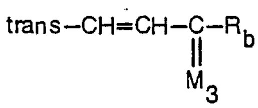 |
| XIV | 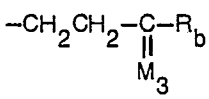 |

CHART C

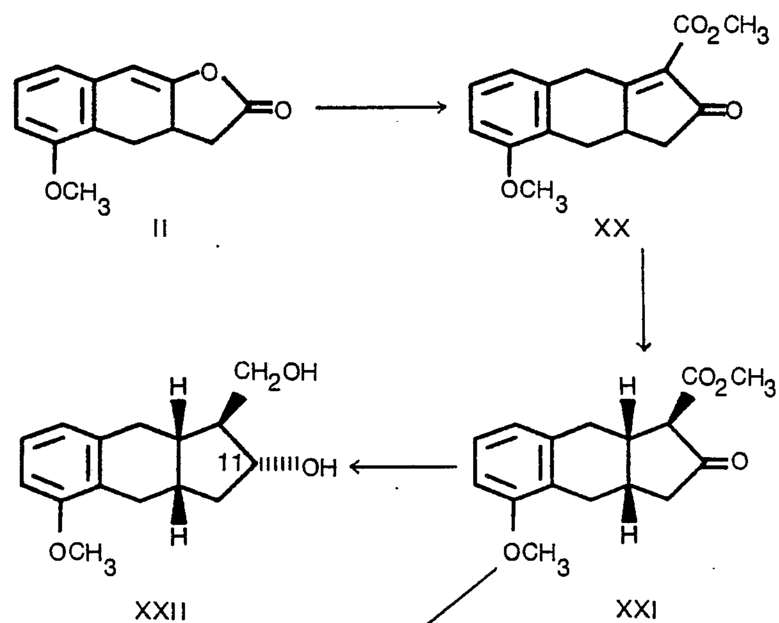

II

XX

XXI

XXII

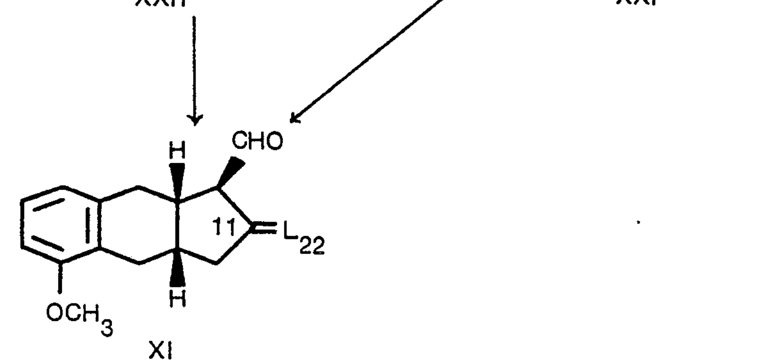

XI

CHART D

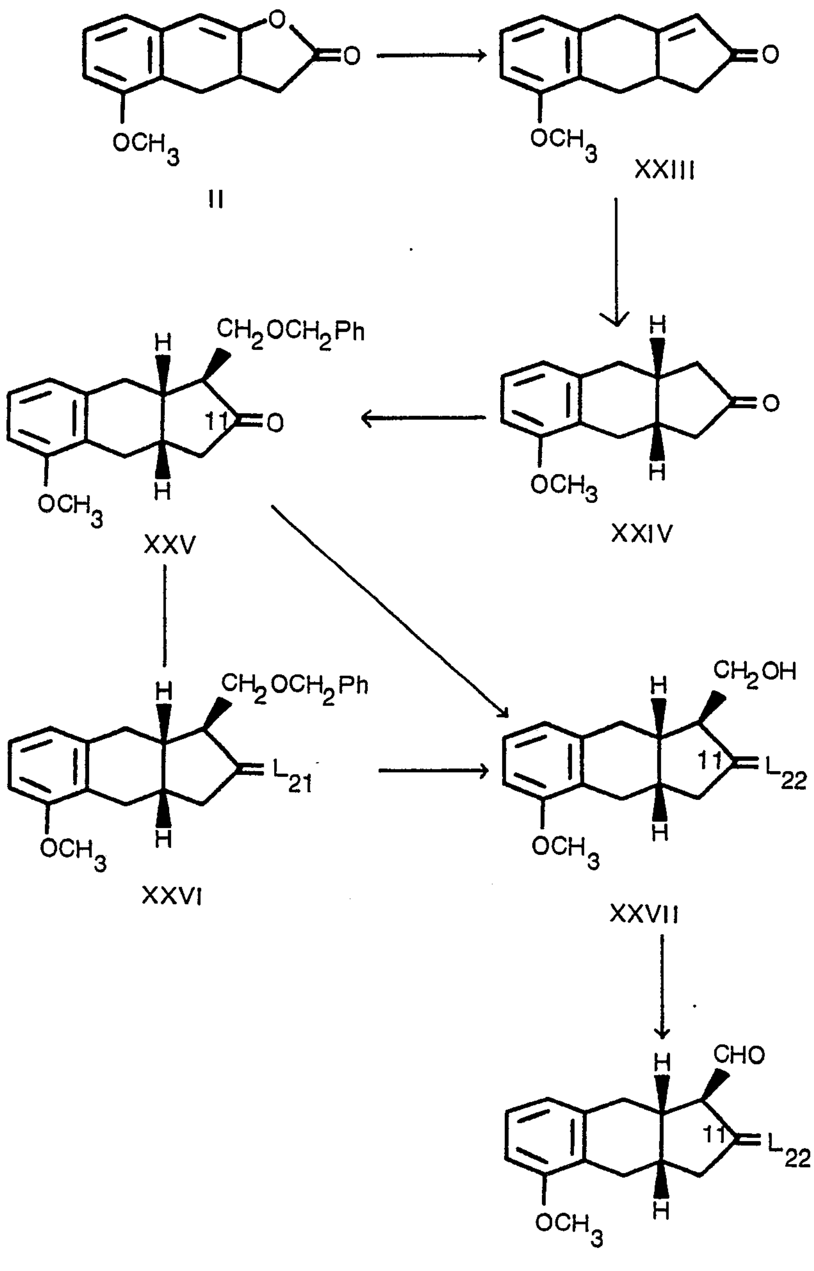

II

XXIII

XXIV

XXV

XXVI

XXVII

XI

CHART E

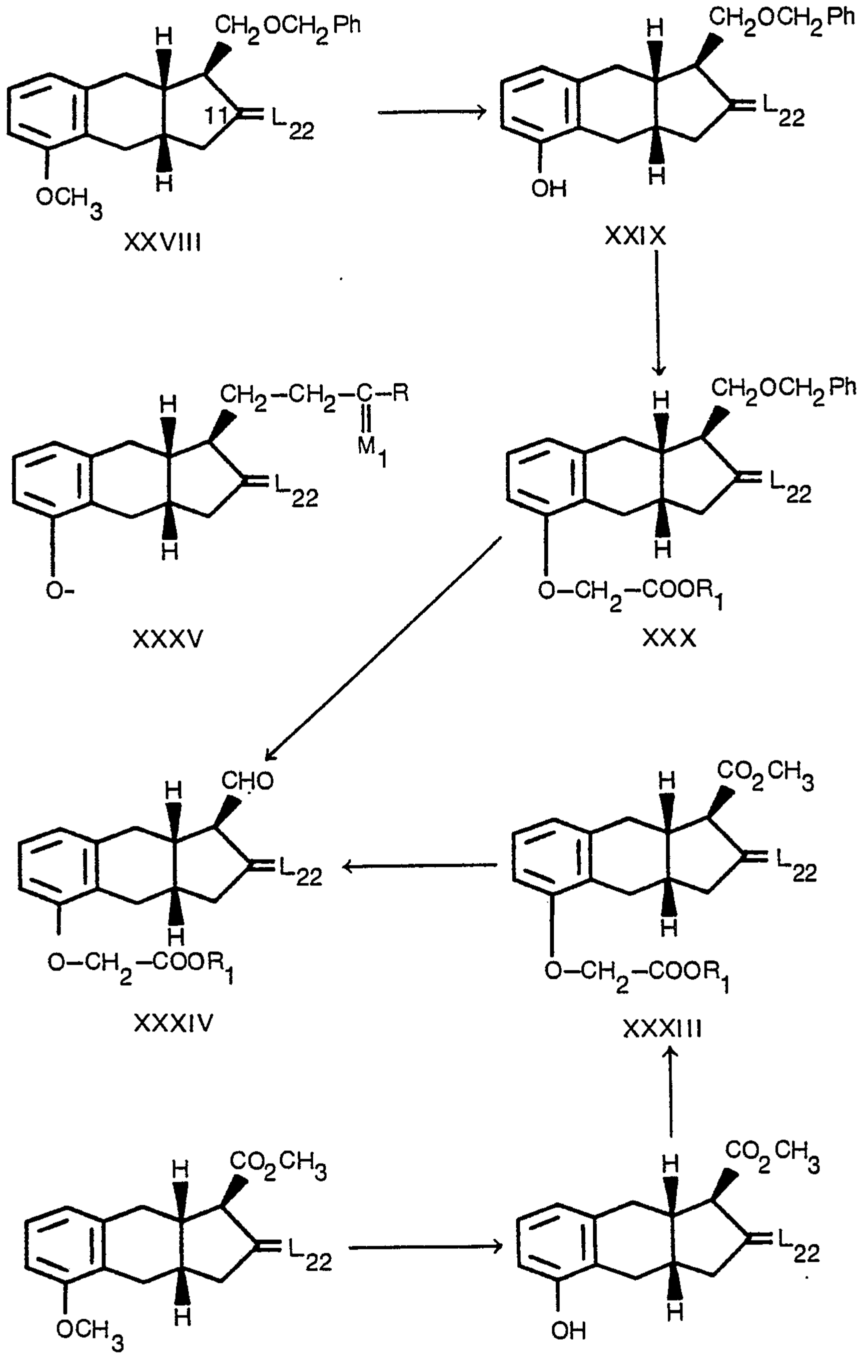

XXVIII

XXIX

XXXV

XXX

XXXIV

XXXIII

XXXI

XXXII

CHART F

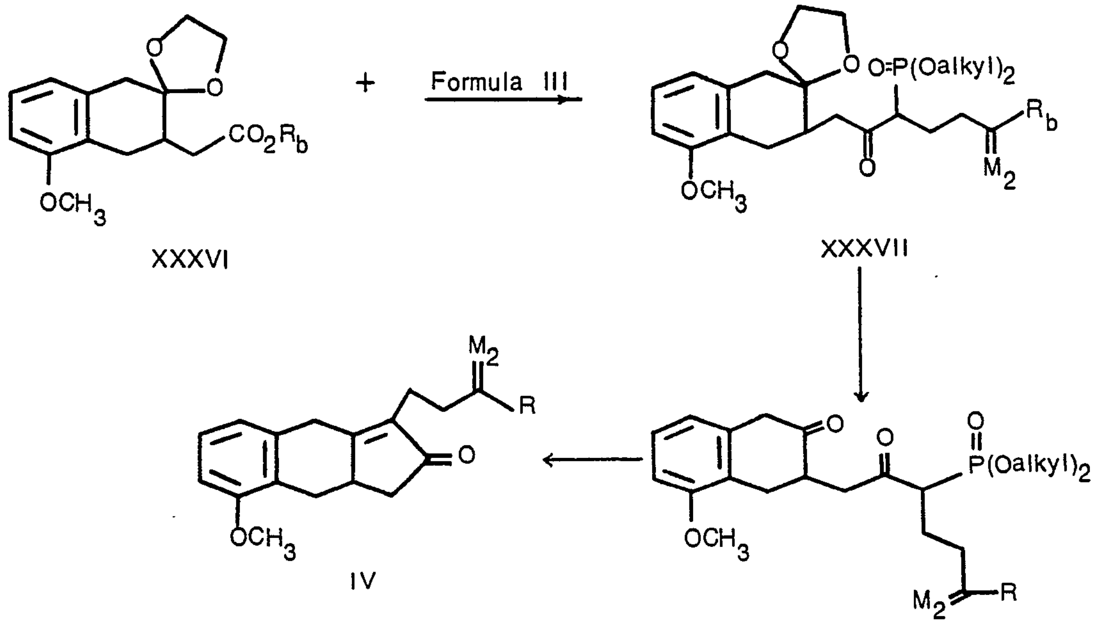


XXXVI

XXXVII

XXXVIII

IV

CHART G

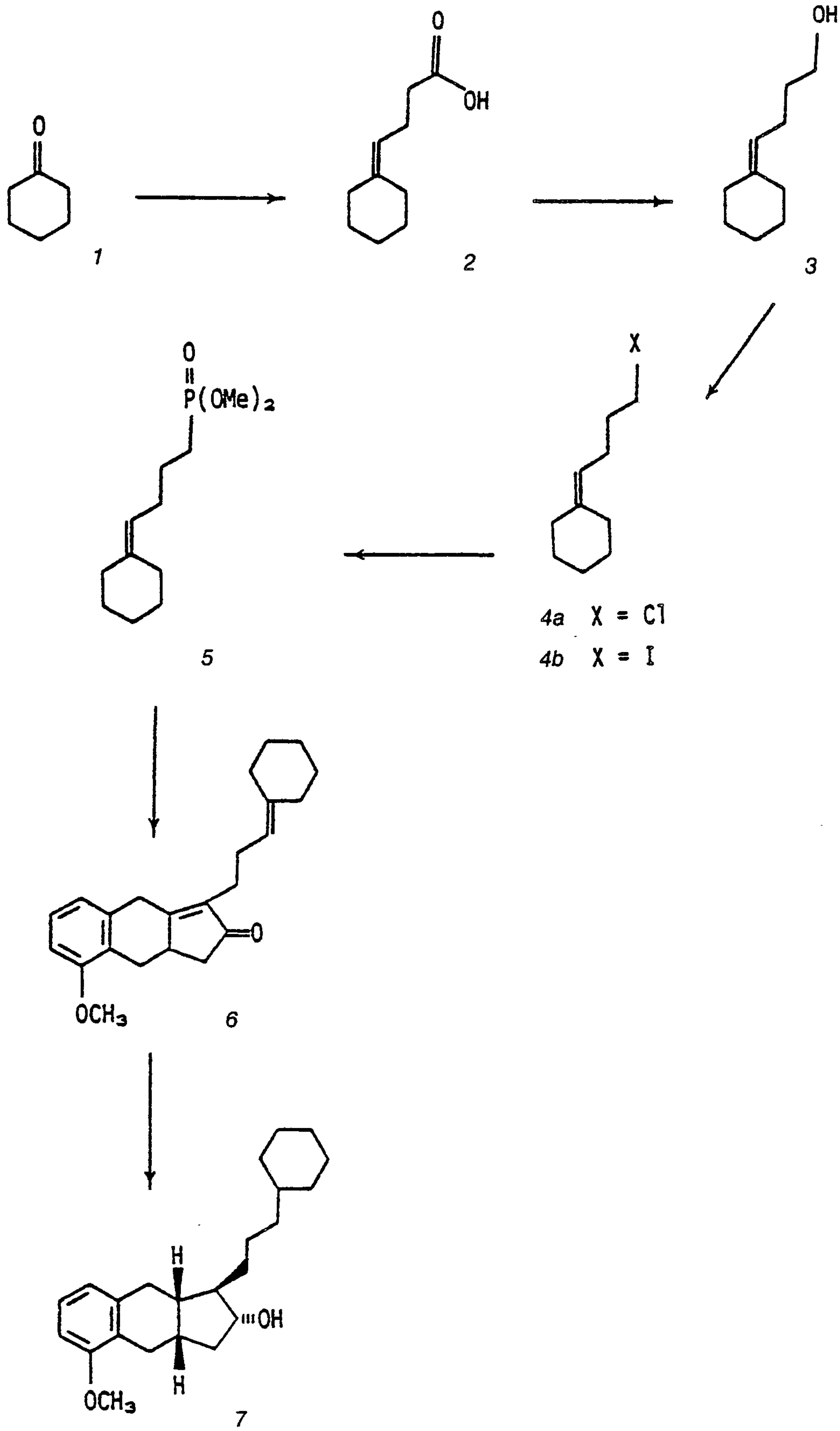

1

2

3

4a X = Cl

4b X = I

5

6

7

CHART H

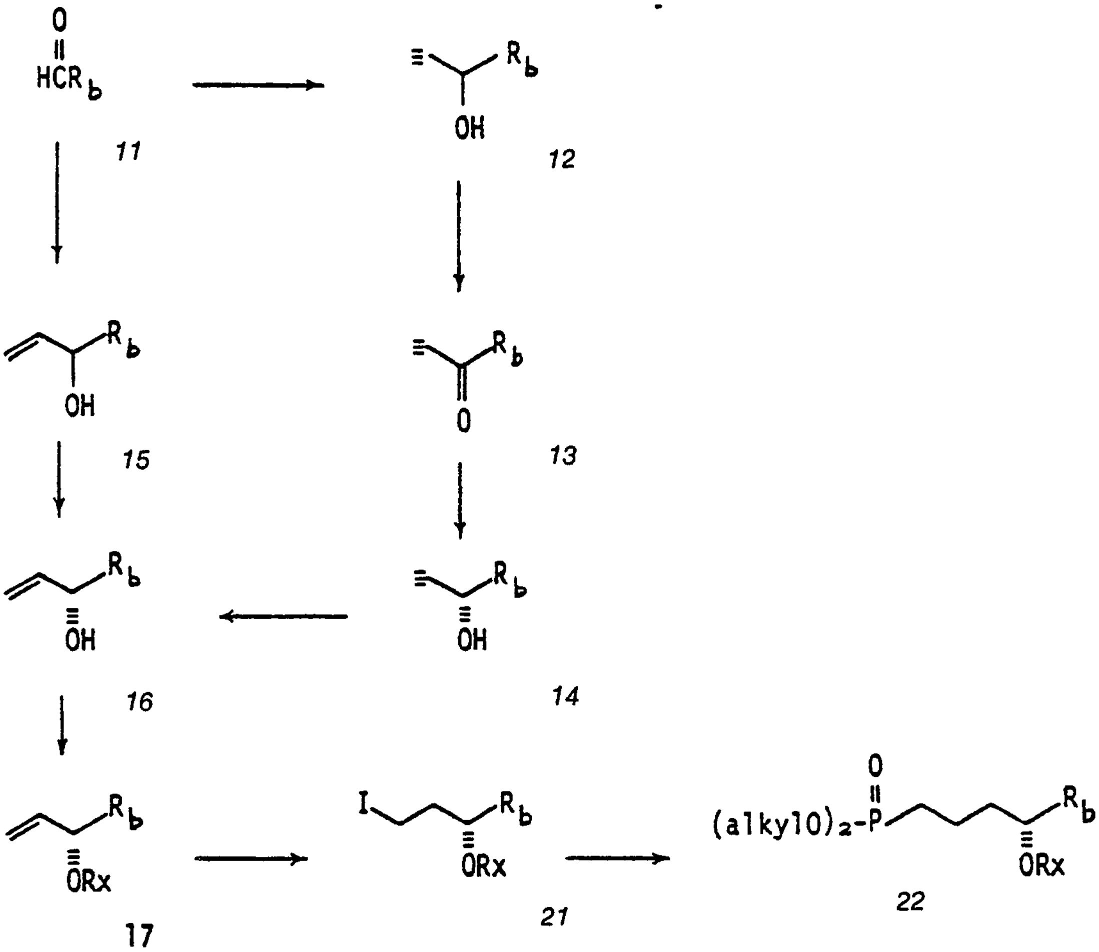

11 12 15 13 16 14 17 21 22

CHART I

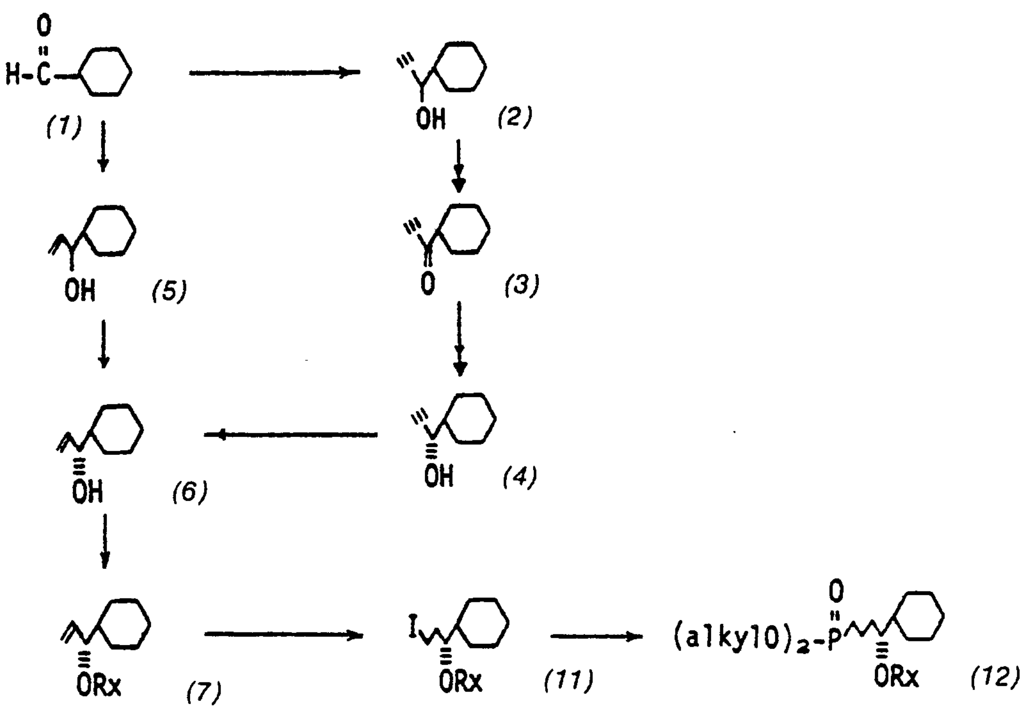

(1) (2) (5) (3) (6) (4) (7) (11) (12)

**Claims**

1. A compound of the formula

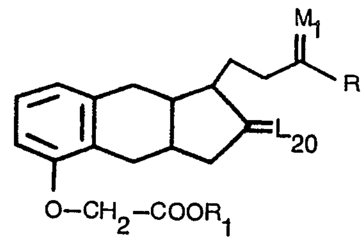

(I)

wherein $R_1$ is H, a pharmacologically-acceptable cation, $C_{1-12}$ alkyl, $C_{3-10}$ cycloalkyl, $C_{7-12}$ aralkyl, phenyl optionally substituted by one, 2 or 3 Cl atoms or $C_{1-3}$ alkyl groups, or phenyl para-substituted by a substituent selected from $—NHCOR_{25}$, $—COR_{26}$, $—OOC—R_{54}$ and $—CH=N—NH—CONH_2$ wherein $R_{25}$ is methyl, phenyl acetamidophenyl, benzamidophenyl or $NH_2$, $R_{26}$ is methyl, phenyl, methoxy or $NH_2$, and $R_{54}$ is phenyl or acetamidophenyl;

$L_{20}$ is α-OH:β-OH, H:H, α-$CH_3$:β-H, α-$CH_2OH$:β-H, O or $CH_2$;

$M_1$ is α-H:β-H, O, α-OH:β-$R_5$ or α-$R_5$:β-OH, $R_5$ being H or $CH_3$; and

R is $C_{4-7}$ cycloalkyl, 4-oxocyclohexyl, 4-tetrahydropyranyl or 2-tetrahydropyranyl;

provided always that $M_1$ is H:H or O and/or $L_{20}$ is α-$CH_3$:β-H, O or $CH_2$.

2. A compound of the formula

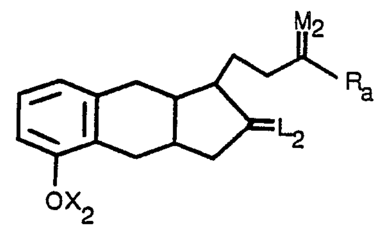

Formula I (e)

wherein $L_{22}$ is α-$OR_x$:β-H, α-H:β-$OR_x$, H:H, α-$CH_3$:β-H, α-$CH_3$:β-H, O or $CH_2$; $R_a$ is $C_{4-7}$ cycloalkyl, 4-(ethylenedioxy)cyclohexyl, 4-oxocyclohexyl, 4-tetrahydropyranyl or 2-tetrahydropyranyl; $X_2$ is H or $CH_3$; and $M_2$ is H:H, ethylenedioxy, α-H:β-$OR_x$ or α-$OR_x$:β-H, $R_x$ being a protecting group;

provided always that $M_2$ is H:H and/or $L_{20}$ is α-$CH_3$:β-H, O or $CH_2$.

3. A compound of the formula

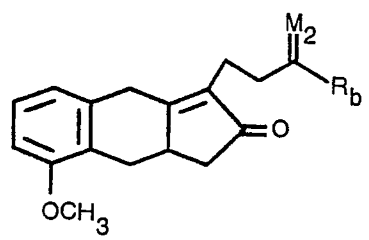

Formula I (d)

wherein $M_2$ is as defined in claim 2 and $R_b$ is $C_{4-7}$ cycloakyl, 4-(ethylenedioxy)cyclohexyl, 4-tetrahydropyranyl or 2-tetrahydropyranyl.

4. A compound of claim 1, wherein $M_1$ is α-OH:β-H or H:H.

5. A compound of claim 1, wherein $M_1$ is H:H.

6. A compound of claim 1 or claim 2, wherein $L_{20}$ is α-OH:β-H and $M_1$ or $M_2$ is H:H.

7. A compound of claim 1 or claim 2, wherein $L_{20}$ is α-$CH_3$:β-H and $M_1$ is α-OH:β-H or $M_2$ is α-$OR_x$:β-H.

8. A compound of any preceding claim, wherein R is cyclohexyl.

9. A compound of claim 1, which is 15-cyclohexyl-9,15-dideoxy-13,14-dihydro-2′,9α-methano-3-oxa-4,5,6,16,17,18,19,20-octanor-3,7-(1′,3′-interphenylene)-$PGF_1$;

15-cyclohexyl-9,15-dideoxy-13,14-dihydro-16(RS)-hydroxy-2′,9α-methano-3-oxa-4,5,6,16,17,18,19,20-octanor-3,7-(1′,3′-interphenylene)-$PGF_1$;

(11RS,15R)-15-cyclohexyl-9,11-dideoxy-13,14-dihydro-2′,9α-methano-3-oxa-4,5,6,16,17,18,19,20-octanor-3,7-(1′,3′-interphenylene)-$PGF_1$; or

(15R)-15-cyclohexyl-9,11-dideoxy-13,14-dihydro-2′,9α-methano-3-oxa-4,5,6,16,17,18,19,20-octanor-3,7-(1′,3′-interphenylene)-$PGF_1$; or

a salt or isomer thereof.

**Patentansprüche**

1. Eine Verbindung der Formel

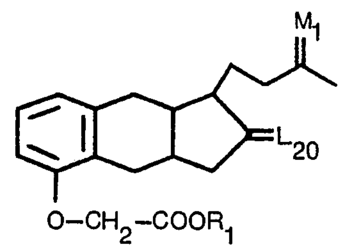

(I)

wobei $R_1$ H, ein pharmakologisch annehmbares Kation, $C_{1-12}$ Alkyl, $C_{1-10}$ Cycloalkyl, $C_{7-12}$ Aralkyl, wahlweise durch ein, 2 oder 3 Cl-Atome oder $C_{1-3}$ Alkyl-Gruppen substituiertes Phenyl oder durch einen aus der Gruppe $—NHCOR_{25}$, $—COR_{26}$, $—OOC—R_{54}$ und $—CH{=}N—NH—CONH_2$ ausgewählten Substituenten parasubstituiertes Phenyl ist, wobei $R_{25}$ Methyl, Phenylacetamidophenyl, Benzamidophenyl oder $NH_2$, $R_{26}$ Methyl, Phenyl, Methoxy oder $NH_2$ und $R_{54}$ Phenyl oder Acetamidophenyl ist;

$L_{20}$ α-OH:β-H, α-H:β-OH, H:H, α-$CH_3$:β-H, α-$CH_2OH$:β-H, O oder $CH_2$ ist;

$M_1$ α-H:β-H, O, α-OH:β-$R_5$ oder α-$R_5$:β-OH ist, wobei $R_5$ H oder $CH_3$ ist; und

R $C_{4-7}$ Cycloalkyl, 4-Oxocyclohexyl, 4-Tetrahydropyranyl oder 2-Tetrahydropyranyl ist;

Stets inter der Voraussetzung, daß $M_1$ H:H oder O und/oder $L_{20}$ α-$CH_3$:β-H, O oder $CH_2$ ist.

2. Eine Verbindung der Formel

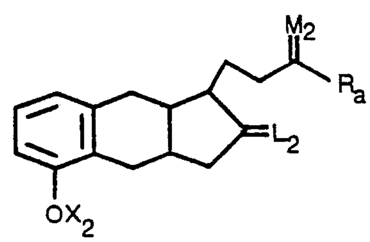

Formel I(e)

worin $L_{22}$ α-$OR_x$:β-H, α-H:β-$OR_x$, H:H, α-$CH_3$:β-H, α-$CH_3$:β-H, O oder $CH_2$ ist; $R_a$ $C_{4-7}$ Cycloalkyl, 4-(Äthylendioxy)Cyclohexyl, 4-Oxocyclohexyl, 4-Tetrahydropyranyl oder 2-Tetrahydropyranyl ist; $X_2$ H oder $CH_3$ ist; und $M_2$ H:H Äthylendioxy, α-H:β-$OR_x$ oder α-$OR_x$:β-H ist, wobei $R_x$ eine schützende Gruppe ist;

stets unter der Voraussetzung, daß $M_2$ H:H und/oder $L_{20}$ α-$CH_3$:β-H, O oder $CH_2$ ist.

3. Eine Verbindung der Formel

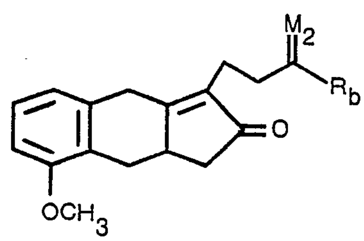

Formel I(d).

worin $M_2$ wie in Anspruch 2 definiert und $R_b$ $C_{4-7}$ Cycloalkyl, 4-(Äthylendioxy)Cyclohexyl, 4-Tetrahydropyranyl oder 2-Tetrahydropyranyl ist.

4. Eine Verbindung nach Anspruch 1, worin $M_1$ α-OH:β-H oder H:H ist.

5. Eine Verbindung nach Anspruch 1, worin $M_1$ H:H ist.

6. Eine Verbindung nach Anspruch 1 oder Anspruch 2, worin $L_{20}$ α-OH:β-H und $M_1$ oder $M_2$ H:H ist.

7. Eine Verbindung nach Anspruch 1 oder Anspruch 2, worin $L_{20}$ α-$CH_3$:β-H und $M_1$ α-OH:β-H oder $M_2$ α-$OR_x$:β-H ist.

8. Eine Verbindung nach einem der vorstehenden Ansprüche, worin R Cyclohexyl ist.

9. Eine Verbindung nach Anspruch 1, die

15-Cyclohexyl-9,15-Dideoxy-13,14-Dihydro-2′,9α-Methano-3-Oxa-4,5,6,16,17,18,19,20-Octanor-3,7-(1′,3′-Interphenylen)-$PGF_1$ ist;

15-Cyclohexyl-9,15-Dideoxy-13,14-Dihydro-16(RS)-Hydroxy-2′,9α-Methano-3-Oxa-4,5,6,16,17,18,19,20-Octanor-3,7-(1′,3′-Interphenylen)-$PGF_1$ ist;

(11RS,15R)-15-Cyclohexyl-9,11-Dideoxy-13,14-Dihydro-2′,9α-Methano-11-Methyl-3-Oxa-4,5,6,16,17,18,19,20-Octanor-3,7-(1′,3′-Interphenylen)-$PGF_1$ ist; oder

(15R)-15-Cyclohexyl-9,11-Dideoxy-13,14-Dihydro-2′,9α-Methano-11-Methylen-4,5,6,16,17,18,19,20-Octanor-3-Oxa-3,7-(1′,3′-Interphenylen)-$PGF_1$ ist; oder

ein Salz oder Isomer davon.

**Revendications**

1. Composé de formule

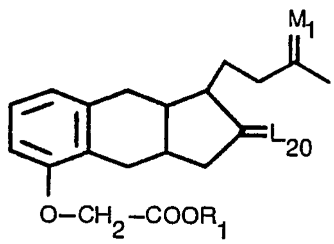

(I)

dans laquelle $R_1$ représente H, un cation pharmacologiquement acceptable, un groupe alkyle en $C_1$ à $C_{12}$, cycloalkyle en $C_3$ à $C_{10}$, aralkyle en $C_7$ à $C_{12}$, phényle facultativement substitué par un, 2 ou 3 atomes de chlore ou groupes alkyle en $C_1$ à $C_3$, ou phényle substitué en para par un substituant choisi parmi les substituants —$NHCOR_{25}$, —$COR_{26}$, —OOC—$R_{54}$ et —CH=N—NH—$CONH_2$ où $R_{25}$ est un radical méthyle, phényle, acétamidophényle, benzamidophényle ou $NH_2$, $R_{26}$ est un groupe méthyle, phényle, méthoxy ou $NH_2$ et $R_{54}$ est un groupe phényle ou acétamidophényle;

$L_{20}$ représente α-OH:β-H, α-H:β-OH, H:H, α-$CH_3$:β-H, α-$CH_2OH$:β-H, O ou 2':

$M_1$ représente α-H:β-H, O, α-OH:β-$R_5$ ou α-$R_5$:β-OH, $R_5$ étant l'hydrogène ou un groupe $CH_3$; et

R est un groupe cycloalkyle en $C_4$ à $C_7$, 4-oxocyclohexyle, 4-tétrahydropyrannyle ou 2-tétrahydropyrannyle;

pourvu toujours que $M_1$ représente H:H ou O et/our que $L_{20}$ représente α-$CH_3$:β-H, O ou $CH_2$.

2. Composé de formule

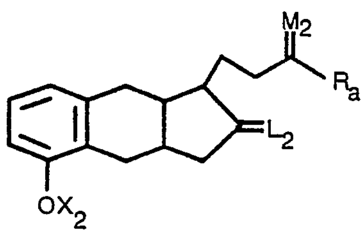

Formule I(e)

dans laquelle $L_{22}$ représente α-$OR_x$:β-H, α-H:β-$OR_x$, H:H, α-$CH_3$:β-H, α-$CH_3$:β-H, O ou $CH_2$; $R_a$ est un groupe cycloalkyle en $C_4$ à $C_7$, 4-(éthylènedioxy)cyclohexyle, 4-oxocyclohexyle, 4-tétrahydropyrannyle ou 2-tétrahydropyrannyle; $X_2$ représente H ou $CH_3$; et $M_2$ représente H:H, un groupe éthylènedioxy, α-H:β-$OR_x$ ou α-$OR_x$:β-H, $R_x$ étant un groupe protecteur;

pourvu toujours que $M_2$ représente H:H et/ou que $L_{20}$ représente α-$CH_3$:β-H, O ou $CH_2$.

3. Composé de formule

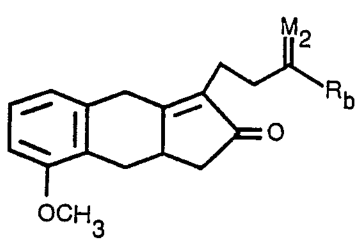

Formule I(d)

dans laquelle $M_2$ est tel que défini dans la revendication 2 et $R_b$ est un groupe cycloalkyle en $C_4$ à $C_7$, 4-(éthylènedioxy)cyclohexyle, 4-tétrahydropyrannyle ou 2-tétrahydropyrannyle.

4. Composé suivant la revendication 1, dans lequel $M_1$ représente α-OH:β-H ou H:H.

5. Composé suivant la revendication 1, dans lequel $M_1$ représente H:H.

6. Composé suivant la revendication 1 ou la revendication 2, dans lequel $L_{20}$ représente α-OH:β-H et $M_1$ ou $M_2$ représente H:H.

7. Composé suivant la revendication 1 ou la revendication 2, dans lequel $L_{20}$ représente α-$CH_3$:β-H et $M_1$ représente α-OH:β-H ou $M_2$ représente α-$OR_x$:β-H.

8. Composé suivant l'une quelconque des revendications précédentes, dans lequel R est un groupe cyclohexyle.

9. Composé suivant la revendication 1, qui est le 15-cyclohexyl-9,15-didéoxy-13,14-dihydro-2',9α-méthano-3-oxa-4,5,6,16,17,18,19,20-octanor-3,7-(1',3'-interphénylene)-$PGF_1$;

le 15-cyclohexyl-9,15-didéoxy-13,14-dihydro-16(RS)-hydroxy-2',9α-méthano-3-oxa-4,5,6,16,17,18,19,20-octanor-3,7-(1',3'-interphénylene)-$PGF_1$;

le (11RS,15R)-15-cyclohexyl-9,11-didéoxy-13,14-dihydro-2',9α-méthano-11-méthyl-3-oxa-4,5,6,16,17,18,19,20-octanor-3,7-(1',3'-interphénylene)-PG$F_1$; ou

le (15R)-15-cyclohexyl-9,11-didéoxy-13,14-dihydro-2',9α-méthano-11-méthylène-4,5,6,16,17,18,19,20-octanor-3-oxa-3,7-(1',3'-interphénylene)-PG$F_1$; ou

un sel ou isomère de ce composé.